# EUROPEAN PATENT APPLICATION

(11) **EP 4 711 355 A1**
(43) Date of publication of application: **18.03.2026**
(21) Application number: 24803084.3
(22) Date of filing: 10.05.2024
(51) Int. Cl.: C07D 207/08, A61K 31/40, A61P 9/00

(54) **CRYSTAL FORM OF LIPOPROTEIN COMPOUND AND PREPARATION METHOD THEREFOR**

(30) Priority: 11.05.2023 CN 202310529962; 25.03.2024 CN 202410340638
(71) Applicant: Jiangsu Hengrui Pharmaceuticals Co., Ltd., Lianyungang, Jiangsu 222047 (CN)
(72) Inventor: YAO, Jiaqi, Lianyungang, Jiangsu 222047 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: Jones Day
(86) International application number: PCT/CN2024/092233
(87) International publication number: WO 2024/230805

(57) **Abstract**

The present disclosure relates to a crystal form of a lipoprotein compound and a preparation method therefor. Specifically, the present disclosure provides a crystal form of (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid and a preparation method therefor.

## Description

### TECHNICAL FIELD

The present disclosure pertains to the field of pharmaceuticals, and relates to a crystal form of a lipoprotein compound and a preparation method therefor.

### BACKGROUND

Lipoprotein (a) [Lp(a)] is a low-density lipoprotein-like blood lipid particle, mainly composed of a core rich in cholesterol esters and a unique apolipoprotein (a) [Apo(a)]. It features genetic polymorphism and long-term stability, and shows a skewed distribution in the population. Studies have found that elevated Lp(a) is associated with an increased risk of cardiovascular events and related revascularization.

PCT/CN2022/129479 discloses a new type of lipoprotein compound, namely (2S)-3-(3- { [(2- {3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid (compound A).

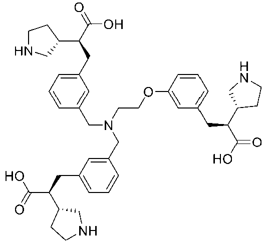

The crystal structure of a pharmaceutically active ingredient often affects the chemical and physical stability of the drug. Different crystallization and storage conditions may lead to changes in the crystal structure of the compound, sometimes accompanied by the production of other forms of crystal structures. Generally speaking, amorphous drug products do not have a regular crystal structure and often have other defects, such as poor product stability, difficulty in filtration, easy agglomeration, and poor fluidity. Therefore, studying their crystal form is of great significance for developing drugs that are suitable for industrial production and have good biological activity.

On the other hand, salt formation can improve some undesirable physicochemical or biological properties of drugs. It is of great significance to develop a salt that has more superior physical and chemical or pharmaceutical properties compared to (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid. Given the significance of solid drug crystal forms and their stability in their clinical applications, in-depth research on the polymorphs of pharmaceutically acceptable salts of the compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid is also of great significance for the development of drugs suitable for industrial production and having good biological activity.

### SUMMARY OF THE INVENTION

In an aspect, the present disclosure provides a crystal form A of compound (2S)-3-(3- { [(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 8.343, 18.020, and 21.028.

In some embodiments, the crystal form A has an X-ray powder diffraction pattern represented by a diffraction angle 2*θ* having characteristic peaks at 8.343, 12.559, 15.612, 18.020, and 21.028.

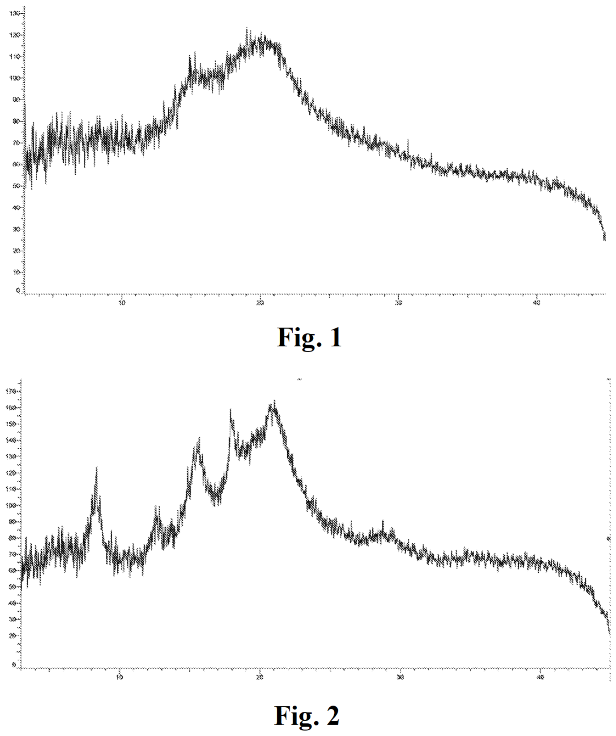

In other embodiments, the crystal form A has an X-ray powder diffraction pattern represented by a diffraction angle 2*θ* as shown in Fig. 2.

In an aspect, the present disclosure provides a crystal form B of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 7.794, 15.530, 19.456, 20.559, and 21.500.

In some embodiments, the crystal form B has an X-ray powder diffraction pattern represented by a diffraction angle 2*θ* having characteristic peaks at 7.794, 11.832, 15.530, 19.456, 20.559, 21.500, and 29.097.

In some embodiments, the crystal form B has an X-ray powder diffraction pattern represented by a diffraction angle 2*θ* having characteristic peaks at 7.794, 11.832, 12.642, 15.530, 19.456, 20.559, 21.500, and 29.097.

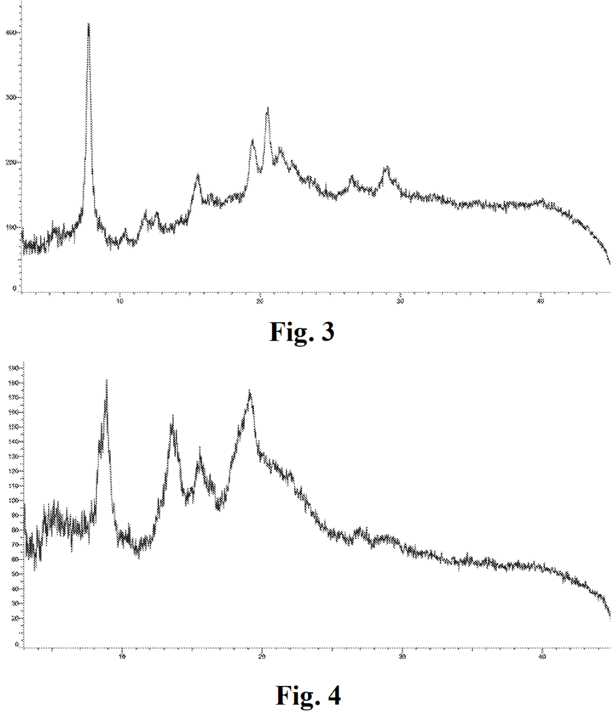

In other embodiments, the crystal form B has an X-ray powder diffraction pattern represented by a diffraction angle 2*θ* as shown in Fig. 3.

In an aspect, the present disclosure provides a crystal form C of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 8.547, 8.847, 13.567, 15.576, and 19.169.

In some embodiments, the crystal form C has an X-ray powder diffraction pattern represented by a diffraction angle 2*θ* having characteristic peaks at 8.547, 8.847, 10.487, 13.567, 15.576, and 19.169.

In some embodiments, the crystal form C has an X-ray powder diffraction pattern represented by a diffraction angle 2*θ* having characteristic peaks at 8.547, 8.847, 10.487, 13.567, 15.576, 19.169, and 21.973.

In other embodiments, the crystal form C has an X-ray powder diffraction pattern represented by a diffraction angle 2*θ* as shown in Fig. 4.

In an aspect, the present disclosure provides a crystal form D of compound (2S)-3-(3- { [(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 13.834, 16.316, and 20.830.

In some embodiments, the crystal form D has an X-ray powder diffraction pattern represented by a diffraction angle 2*θ* having characteristic peaks at 7.928, 13.834, 16.316, and 20.830.

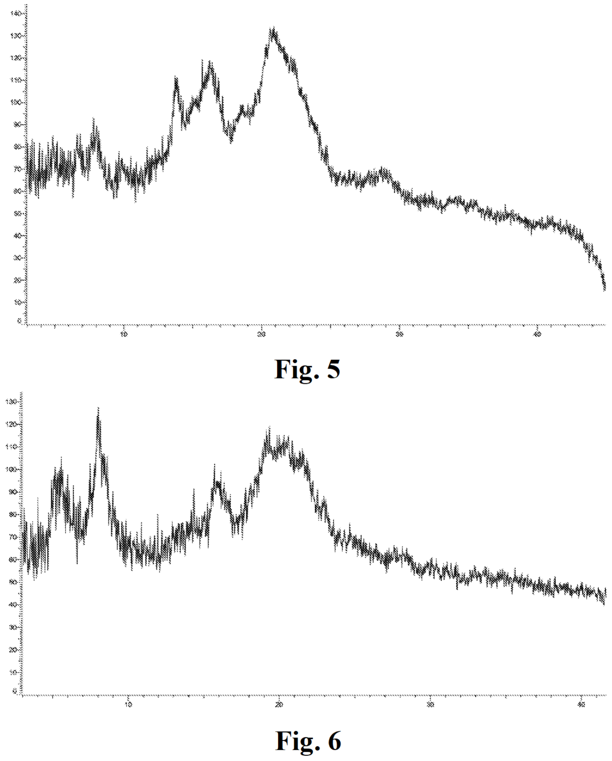

In other embodiments, the crystal form D has an X-ray powder diffraction pattern represented by a diffraction angle 2*θ* as shown in Fig. 5.

In an aspect, the present disclosure provides a crystal form E of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 4.933, 6.545, 8.528, 20.874, and 22.149.

In some embodiments, the crystal form E has an X-ray powder diffraction pattern represented by a diffraction angle 2*θ* having characteristic peaks at 4.671, 4.933, 6.545, 8.528, 16.262, 20.874, and 22.149.

In some embodiments, the crystal form E has an X-ray powder diffraction pattern represented by a diffraction angle 2*θ* having characteristic peaks at 4.671, 4.933, 6.545, 8.528, 13.037, 15.017, 16.262, 20.874, and 22.149.

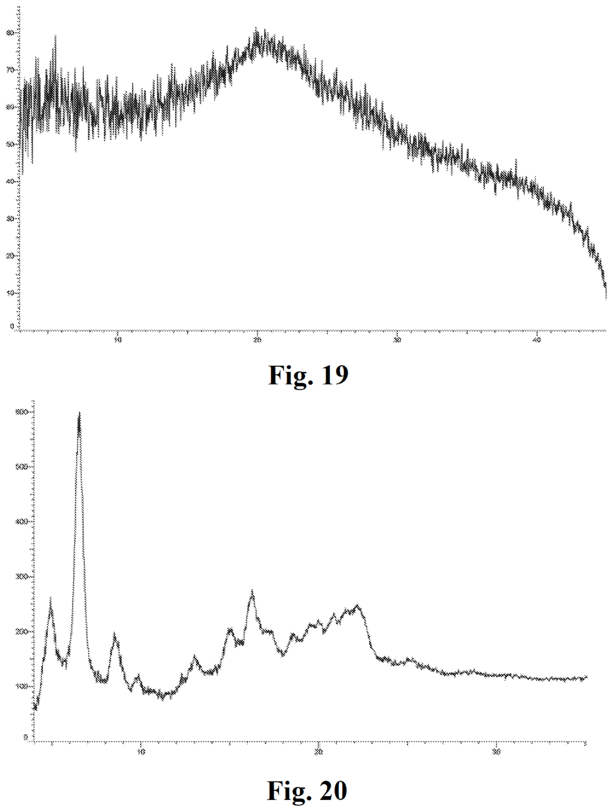

In other embodiments, the crystal form E has an X-ray powder diffraction pattern represented by a diffraction angle 2*θ* as shown in Fig. 20.

Furthermore, the X-ray powder diffraction patterns of the crystal form A, crystal form B, crystal form C, crystal form D, and crystal form E of the compound A disclosed herein are represented by a diffraction angle 2*θ*, wherein the error range of the 2*θ* angle is ±0.2.

In another aspect, the present disclosure further provides a method for preparing the aforementioned crystal forms of compound A, which is selected from any of the following methods,
method 1:
   (a) mixing compound (2S)-3-(3-{[(2-{3-[(2S)-2-carboxyl-2-[(3R)-pyrrolidin-3-yl]ethyl] phenoxy}ethyl)({3-[(2S)-2-carboxyl-2-[(3R)-pyrrolidin-3-yl]ethyl]phenyl}methyl)amino]methyl} phenyl)-2-[(3R)-pyrrolidin-3-yl]propionic acid with a solvent, and stirring or heating the mixture for dissolution,
   (b) crystallization;
or, method 2:
   (a) mixing compound (2S)-3-(3-{[(2-{3-[(2S)-2-carboxyl-2-[(3R)-pyrrolidin-3-yl]ethyl] phenoxy}ethyl)({3-[(2S)-2-carboxyl-2-[(3R)-pyrrolidin-3-yl]ethyl]phenyl}methyl)amino]methyl} phenyl)-2-[(3R)-pyrrolidin-3-yl]propionic acid with a solvent, and stirring or heating the mixture for dissolution,
   (b) adding a second solvent and stirring the mixture;
or, method 3:
   (a) mixing compound (2S)-3-(3-{[(2-{3-[(2S)-2-carboxyl-2-[(3R)-pyrrolidin-3-yl]ethyl] phenoxy}ethyl)({3-[(2S)-2-carboxyl-2-[(3R)-pyrrolidin-3-yl]ethyl]phenyl}methyl)amino]methyl} phenyl)-2-[(3R)-pyrrolidin-3-yl]propionic acid with a solvent,
   (b) stirring the mixture.

In some embodiments, the method for preparing the aforementioned crystal forms of compound A according to the present disclosure further comprises any one of the steps of crystallization, filtration, washing or drying.

In some embodiments, the crystallization includes but is not limited to stirring crystallization (antisolvent crystallization, pulping crystallization) and volatilization crystallization.

In some embodiments, the drying method includes but is not limited to forced air drying and vacuum drying. The drying temperature is generally 25°C to 100°C, preferably 30°C to 70°C, such as 40°C, 50°C or 60°C.

In some embodiments, the method for preparing the crystal form C of the compound as described above comprises (a) mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with one or more solvents including water, isopropanol, tetrahydrofuran, ethanol, acetone, and ethyl acetate, (b) pulping crystallization, filtration and drying.

In some embodiments, the method for preparing the crystal form E of the compound as described above comprises (a) mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid with one of solvents including methanol, ethyl acetate/ethanol (v/v = 1:1), and acetonitrile/methanol (v/v = 1:1)., (b) pulping crystallization, filtration and drying.

In another aspect, the present disclosure further provides a pharmaceutically acceptable salt of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid.

In some embodiments, the pharmaceutically acceptable salt is selected from hydrochloride, succinate, meglumine salt, maleate, hippurate, sulfate, phosphate, methanesulfonate, tartrate, fumarate, citrate, sodium salt, potassium salt and arginine salt.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to acid molecules or base molecules is 1:0.2 to 1:5, and preferably 1:0.5, 1:1, 1:2, 1:3 or 1:4.

In some embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to acid molecules is 1:1 to 1:4.

In some embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to base molecules is 1:1 to 1:3.

Some embodiments provide a hydrochloride of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid, wherein the chemical ratio of the compound to HCl is 1:1 to 1:3.

Some embodiments provide a maleate of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid, wherein the chemical ratio of the compound to HCl is 1:1.

Some embodiments provide an L-tartarate of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid, wherein the chemical ratio of the compound to L-tartaric acid is 1:1.

Some embodiments provide a maleate of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid, wherein the chemical ratio of the compound to maleic acid is 1:1.

Some embodiments provide a meglumine salt of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid, wherein the chemical ratio of the compound to meglumine is 1:1 to 1:2.

The present disclosure further provides a method for preparing the aforementioned pharmaceutically acceptable salt, comprising a step of forming a salt of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with an acid or a base.

In some embodiments, the acid used in the salt-forming reaction is selected from, but not limited to, HCl, succinic acid, maleic acid, hippuric acid, sulfuric acid, phosphoric acid, methanesulfonic acid, tartaric acid, fumaric acid, and citric acid.

In some embodiments, the base used in the salt-forming reaction is selected from, but not limited to, meglumine salt, sodium hydroxide, potassium hydroxide, and arginine.

In some embodiments, the solvent used in the salt-forming reaction is selected from at least one of ethanol, ethyl acetate, tetrahydrofuran, acetonitrile, acetone, methanol, 1,4-dioxane, isopropanol, 2-methyltetrahydrofuran, ethylene glycol, and water.

In some embodiments, the method for salt-forming according to the present disclosure further comprises any one of the steps of filtration, washing or drying.

The present disclosure provides a potassium salt crystal form α of compound (2S)-3-(3-{ [(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 5.544, 8.016, and 19.356.

In some embodiments, the potassium salt crystal form α has an X-ray powder diffraction pattern represented by a diffraction angle 2*θ* having characteristic peaks at 5.544, 8.016, 19.356, and 21.537.

In other embodiments, the potassium salt crystal form α has an X-ray powder diffraction pattern represented by a diffraction angle 2*θ* as shown in Fig. 6.

The present disclosure further provides a method for preparing the potassium salt crystal form α of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid with ethanol, and (b) adding an aqueous potassium hydroxide solution and stirring the mixture.

The present disclosure provides an arginine salt crystal form α of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 8.191, 10.598, 15.844, 18.447, and 20.119.

In some embodiments, the arginine salt crystal form a has an X-ray powder diffraction pattern represented by a diffraction angle 2*θ* having characteristic peaks at 8.191, 10.598, 15.844, 18.447, 20.119, and 24.226.

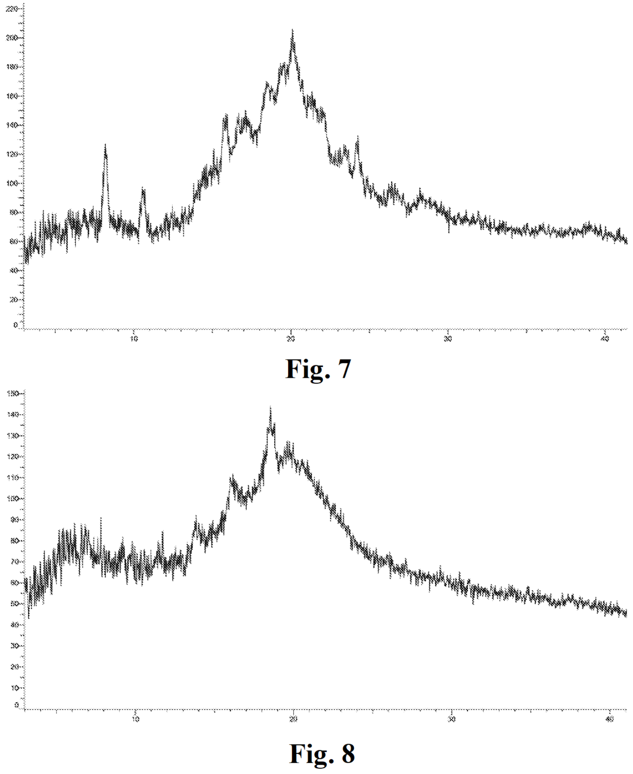

In other embodiments, the arginine salt crystal form a has an X-ray powder diffraction pattern represented by a diffraction angle 2*θ* as shown in Fig. 7.

The present disclosure further provides a method for preparing the arginine salt crystal form a of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with 10% water/tetrahydrofuran, and (b) adding an aqueous arginine solution and stirring the mixture.

The present disclosure further provides a sodium salt crystal form I of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 13.831 and 18.556.

In some embodiments, the sodium salt crystal form I has an X-ray powder diffraction pattern represented by a diffraction angle 2*θ* having characteristic peaks at 7.834, 13.831, 16.194, and 18.556.

In other embodiments, the sodium salt crystal form I has an X-ray powder diffraction pattern represented by a diffraction angle 2*θ* as shown in Fig. 8.

The present disclosure further provides a method for preparing the sodium salt crystal form I of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with 10% water/tetrahydrofuran, and (b) adding a sodium hydoxide solution and stirring the mixture.

The present disclosure further provides a meglumine salt crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid having an X-ray powder diffraction pattern represented by a diffraction angle 2*θ* having characteristic peaks at 5.361, 5.879, 7.118, 8.069, 8.827, and 19.069.

In some embodiments, the meglumine salt crystal form a has an X-ray powder diffraction pattern represented by a diffraction angle 2*θ* having characteristic peaks at 5.361, 5.879, 7.118, 8.069, 8.827, 9.748, 16.230, and 19.069.

In some embodiments, the meglumine salt crystal form a has an X-ray powder diffraction pattern represented by a diffraction angle 2*θ* having characteristic peaks at 5.361, 5.879, 7.118, 8.069, 8.827, 9.748, 13.795, 16.230, 19.069, 21.864, and 26.223.

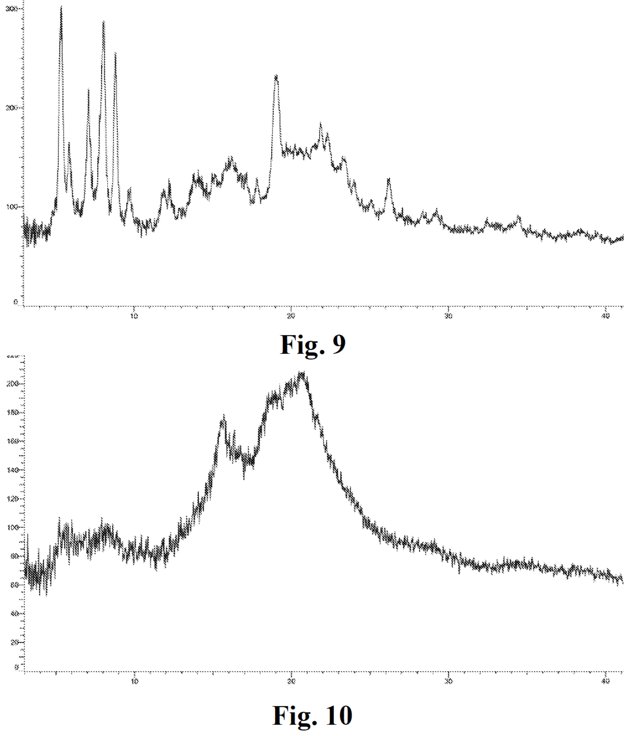

In other embodiments, the meglumine salt crystal form a has an X-ray powder diffraction pattern represented by a diffraction angle 2*θ* as shown in Fig. 9.

The present disclosure further provides a method for preparing the meglumine salt crystal form a of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with ethanol, and (b) adding an aqueous meglumine solution and stirring the mixture.

The present disclosure further provides a maleate crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 7.907, 15.612, 19.174, and 20.701.

In other embodiments, the maleate crystal form a has an X-ray powder diffraction pattern represented by a diffraction angle 2*θ* as shown in Fig. 10.

The present disclosure further provides a method for preparing the maleate crystal form a of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with 10% water/tetrahydrofuran, and (b) adding an aqueous maleic acid solution and stirring the mixture.

The present disclosure further provides a hippurate crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 5.551, 9.410, 13.522, 14.534, 19.956, 21.337, and 22.945.

In some embodiments, the hippurate crystal form a has an X-ray powder diffraction pattern represented by a diffraction angle 2*θ* having characteristic peaks at 5.551, 9.410, 11.209, 13.522, 14.175, 14.534, 19.956, 21.022, 21.337, and 22.945.

In some embodiments, the hippurate crystal form a has an X-ray powder diffraction pattern represented by a diffraction angle 2*θ* having characteristic peaks at 5.551, 9.410, 11.209, 13.522, 14.175, 14.534, 16.709, 19.956, 21.022, 21.337, and 22.945.

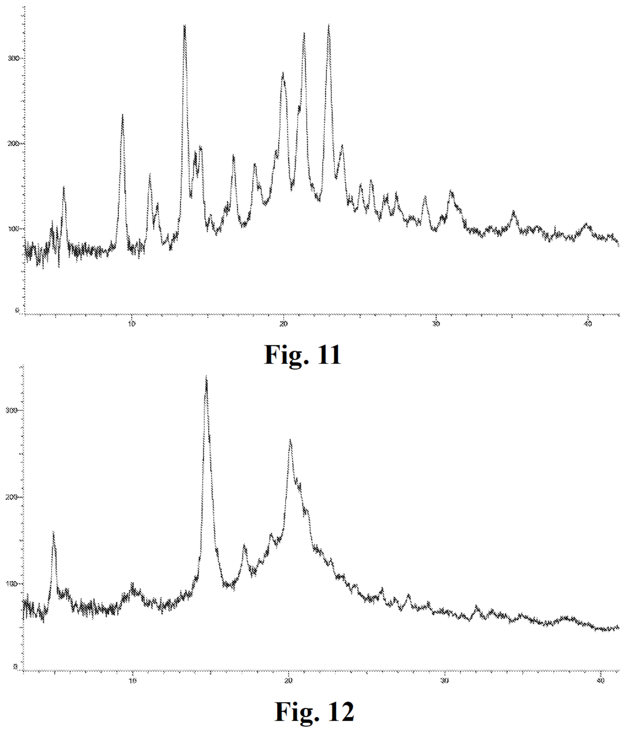

In other embodiments, the hippurate crystal form a has an X-ray powder diffraction pattern represented by a diffraction angle 2*θ* as shown in Fig. 11.

The present disclosure further provides a method for preparing the hippurate crystal form a of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with ethyl acetate, and (b) adding an aqueous hippuric acid solution and stirring the mixture.

The present disclosure further provides a hippurate crystal form β of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 4.944, 14.737, 17.143, and 20.109.

In some embodiments, the hippurate crystal form β has an X-ray powder diffraction pattern represented by a diffraction angle 2*θ* having characteristic peaks at 4.944, 9.978, 14.737, 17.143, and 20.109.

In other embodiments, the hippurate crystal form β has an X-ray powder diffraction pattern represented by a diffraction angle 2*θ* as shown in Fig. 12.

The present disclosure further provides a method for preparing the hippurate crystal form β of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with ethyl acetate, and (b) adding an aqueous hippuric acid solution, and stirring and drying the mixture. The present disclosure further provides a hydrochloride crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid having an X-ray powder diffraction pattern represented by a diffraction angle 2*θ* having characteristic peaks at 6.385, 13.533, 16.203, and 21.244.

In some embodiments, the hydrochloride crystal form a has an X-ray powder diffraction pattern represented by a diffraction angle 2*θ* having characteristic peaks at 6.385, 8.530, 13.533, 16.203, and 21.244.

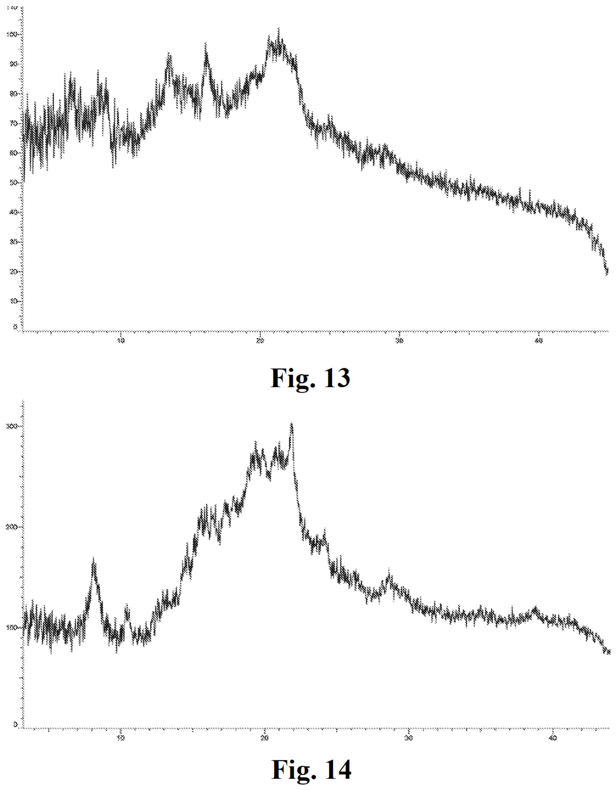

In other embodiments, the hydrochloride crystal form a has an X-ray powder diffraction pattern represented by a diffraction angle 2*θ* as shown in Fig. 13.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to HCl in the hydrochloride crystal form a is 1:1.

The present disclosure further provides a method for preparing the hydrochloride crystal form a of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl }methyl)amino]methyl }phenyl)-2-[(3R)-pyrrolidin-3-yl]propionic acid with solvent (1), the solvent (1) being selected from methanol, ethyl acetate and 1,4-dioxane, and (b) adding an aqueous hydrochloric acid solution and stirring the mixture.

The present disclosure further provides a hydrochloride crystal form b of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid having an X-ray powder diffraction pattern represented by a diffraction angle 2*θ* having characteristic peaks at 8.059, 10.359, 19.412, 20.726, and 21.843.

In some embodiments, the hydrochloride crystal form b has an X-ray powder diffraction pattern represented by a diffraction angle 2*θ* having characteristic peaks at 8.059, 10.359, 16.017, 17.806, 19.412, 20.726, 21.843.

In other embodiments, the hydrochloride crystal form b has an X-ray powder diffraction pattern represented by a diffraction angle 2*θ* as shown in Fig. 14.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to HCl in the hydrochloride crystal form b is 1:1.

The present disclosure further provides a method for preparing the hydrochloride crystal form b of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with acetonitrile, and (b) adding an aqueous hydrochloric acid solution and stirring the mixture.

The present disclosure further provides a hydrochloride crystal form c of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid having an X-ray powder diffraction pattern represented by a diffraction angle 2*θ* having characteristic peaks at 9.395, 11.238, 13.495, and 20.153.

In some embodiments, the hydrochloride crystal form c has an X-ray powder diffraction pattern represented by a diffraction angle 2*θ* having characteristic peaks at 9.395, 11.238, 13.495, 20.153, 21.169, and 23.125.

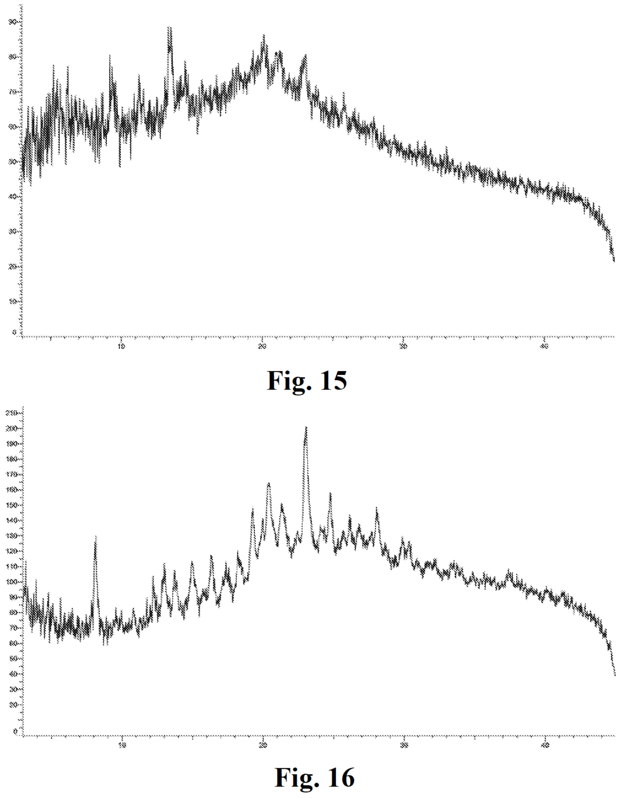

In other embodiments, the hydrochloride crystal form c has an X-ray powder diffraction pattern represented by a diffraction angle 2*θ* as shown in Fig. 15.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to HCl in the hydrochloride crystal form c is 1:1.

The present disclosure further provides a method for preparing the hydrochloride crystal form c of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with 10% water/tetrahydrofuran, and (b) adding an aqueous hydrochloric acid solution and stirring the mixture.

The present disclosure further provides a hydrochloride crystal form d of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid having an X-ray powder diffraction pattern represented by a diffraction angle 2*θ* having characteristic peaks at 8.128, 12.995, 14.962, 16.354, 20.401, and 23.032.

In some embodiments, the hydrochloride crystal form d has an X-ray powder diffraction pattern represented by a diffraction angle 2*θ* having characteristic peaks at 8.128, 12.216, 12.995, 14.962, 16.354, 19.268, 20.401, and 23.032.

In some embodiments, the hydrochloride crystal form d has an X-ray powder diffraction pattern represented by a diffraction angle 2*θ* having characteristic peaks at 8.128, 12.216, 12.995, 14.962, 16.354, 19.268, 19.965, 20.401, 23.032, and 24.742.

In other embodiments, the hydrochloride crystal form d has an X-ray powder diffraction pattern represented by a diffraction angle 2*θ* as shown in Fig. 16.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to HCl in the hydrochloride crystal form d is 1:1.

The present disclosure further provides a method for preparing the hydrochloride crystal form d of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid with 10% water/isopropanol, and (b) adding an aqueous hydrochloric acid solution and stirring the mixture.

The present disclosure further provides a hydrochloride crystal form e of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl] propionic acid having an X-ray powder diffraction pattern represented by a diffraction angle 2*θ* having characteristic peaks at 9.385, 14.145, 19.709, 20.277, and 21.583.

In some embodiments, the hydrochloride crystal form e has an X-ray powder diffraction pattern represented by a diffraction angle 2*θ* having characteristic peaks at 9.385, 14.145, 15.451, 19.709, 20.277, 21.583, and 23.684.

In some embodiments, the hydrochloride crystal form e has an X-ray powder diffraction pattern represented by a diffraction angle 2*θ* having characteristic peaks at 9.385, 14.145, 15.451, 19.709, 20.277, 21.583, 23.187, 23.684, and 24.366.

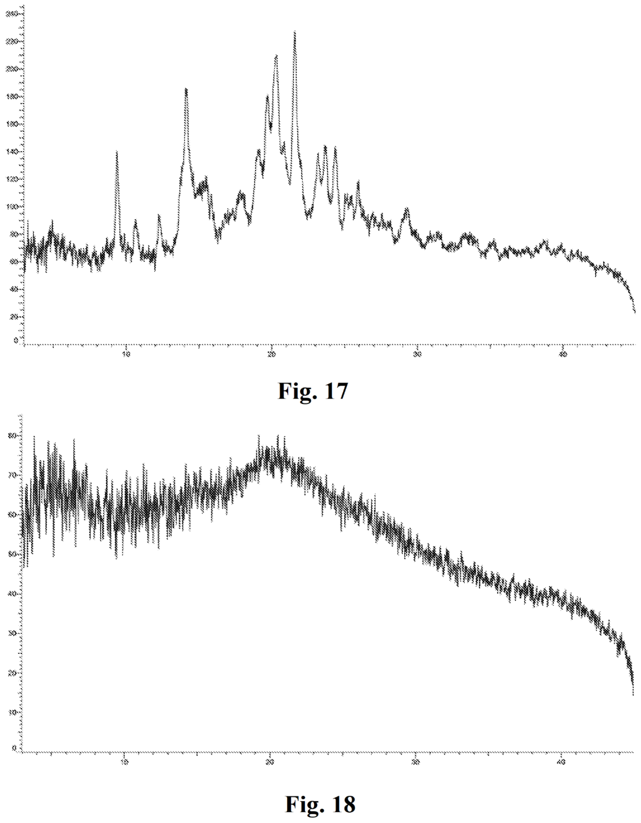

In other embodiments, the hydrochloride crystal form e has an X-ray powder diffraction pattern represented by a diffraction angle 2*θ* as shown in Fig. 17.

In other embodiments, the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid to HCl in the hydrochloride crystal form e is 1:1.

The present disclosure further provides a method for preparing the hydrochloride crystal form e of the compound as described above, which comprises steps of mixing compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl }methyl)amino]methyl }phenyl)-2-[(3R)-pyrrolidin-3-yl]propionic acid with solvent (2), the solvent (2) being selected from 10% water/isopropanol, acetonitrile, 10% water/tetrahydrofuran, methanol, isopropanol, or their mixed solvents, and (b) adding an aqueous hydrochloric acid solution, and stirring and drying the mixture.

Furthermore, the X-ray powder diffraction patterns of the pharmaceutically acceptable salts of the compound A disclosed herein are represented by a diffraction angle 2*θ*, wherein the error range of the 2*θ* angle is ±0.2. In some embodiments, the method for forming crystal forms according to the present disclosure further comprises any one of the steps of filtration (or centrifugal filtration/centrifugation), washing or drying.

In some embodiments, the drying method includes but is not limited to forced air drying and vacuum drying. The drying temperature is generally 25°C to 100°C, preferably 30°C to 70°C, such as 40°C, 50°C or 60°C.

In another aspect, the present disclosure further provides a pharmaceutical composition, which comprises the aforementioned crystal form and a pharmaceutically acceptable excipient.

The present disclosure further provides a pharmaceutical composition, which is prepared from the aforementioned crystal form and a pharmaceutically acceptable excipient.

The present disclosure further provides a method for preparing a pharmaceutical composition, which comprises the step of mixing the aforementioned crystal form with a pharmaceutically acceptable excipient.

The present disclosure further provides use of the aforementioned pharmaceutically acceptable salt, the aforementioned crystal form, or the aforementioned pharmaceutical composition in the preparation of a medicament for preventing and/or treating a disease or condition associated with elevated LP(a) plasma levels.

The present disclosure further provides use of the aforementioned pharmaceutically acceptable salt, the aforementioned crystal form, or the aforementioned pharmaceutical composition in the preparation of a medicament for preventing and/or treating a cardiovascular disease.

The "2*θ* or 2*θ* angle" in the present disclosure refers to the diffraction angle, *θ* is the Bragg angle in ° or degree; the error range of each characteristic peak 2*θ* is ±0.20 (including the case where the number exceeding 1 decimal place is rounded off), specifically -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, and 0.20.

The numerical values in the present disclosure are instrument measurement values or calculated values after instrument measurement, and have a certain degree of error. Generally speaking, ±10% is within the reasonable error range. Of course, the context in which the numerical value is used needs to be considered. For example, the total impurity content, which is a value with an error change of no more than ±10% after measurement, can be ±9%, ±8%, ±7%, ±6%, ±5%, ±4%, ±3%, ±2% or ±1%, preferably ±5%.

The term "differential scanning calorimetry or DSC" described in the present disclosure refers to measuring the temperature difference and heat flow difference between a sample and a reference during heating or maintaining the sample at a constant temperature, in order to characterize all physical and chemical changes associated with thermal effects and obtain phase change information of the sample.

The "pharmaceutically acceptable excipient" described in the present disclosure includes, but is not limited to, any adjuvant, carrier, glidant, sweetener, diluent, preservative, dye/colorant, flavoring agent, surfactant, wetting agent, dispersant, suspending agent, stabilizer, isotonic agent or emulsifier approved by the U.S. Food and Drug Administration for use by humans or livestock animals.

The "pulping" mentioned in the present disclosure refers to a purification method that utilizes the property that a substance has poor solubility in a solvent but impurities have good solubility in the solvent. Pulping purification can remove color, change the crystal form or remove a small amount of impurities.

The crystal forms mentioned in the present disclosure include but are not limited to solvates of compound A, and the solvents include but are not limited to water.

### BRIEF DESCRIPTION OF THE DRAWINGS

Fig. 1 is the XRPD pattern of amorphous form of compound A.
Fig. 2 is the XRPD pattern of crystal form A of compound A.
Fig. 3 is the XRPD pattern of crystal form B of compound A.
Fig. 4 is the XRPD pattern of crystal form C of compound A.
Fig. 5 is the XRPD pattern of crystal form D of compound A. Fig. 6 is the XRPD pattern of potassium salt crystal form α of compound A.
Fig. 7 is the XRPD pattern of arginine salt crystal form a of compound A.
Fig. 8 is the XRPD pattern of sodium salt crystal form I of compound A.
Fig. 9 is the XRPD pattern of meglumine salt crystal form a of compound A.
Fig. 10 is the XRPD pattern of maleate crystal form a of compound A.
Fig. 11 is the XRPD pattern of hippurate crystal form a of compound A.
Fig. 12 is the XRPD pattern of hippurate crystal form β of compound A.
Fig. 13 is the XRPD pattern of hydrochloride crystal form a of compound A.
Fig. 14 is the XRPD pattern of hydrochloride crystal form b of compound A.
Fig. 15 is the XRPD pattern of hydrochloride crystal form c of compound A.
Fig. 16 is the XRPD pattern of hydrochloride crystal form d of compound A.
Fig. 17 is the XRPD pattern of hydrochloride crystal form e of compound A.
Fig. 18 is the XRPD pattern of hydrochloride (1:4) amorphous form of compound A.
Fig. 19 is the XRPD pattern of hydrochloride (1:1) amorphous form of compound A.
Fig. 20 is the XRPD pattern of crystal form E of compound A.

### DETAILED DESCRIPTION OF PREFERRED EMBODIMENTS

The present disclosure is further described in detail through the following examples and experimental examples. These examples and experimental examples are for illustrative purposes only and are not intended to limit the scope of the present disclosure.

Test conditions of the instruments used in the experiments:
The structures of the compounds were determined by nuclear magnetic resonance (NMR) or mass spectrometry (MS). NMR shifts (δ) were given in 10⁻⁶ (ppm). NMR measurements were performed using a Bruker AVANCE-400 nuclear magnetic spectrometer. The solvents used were deuterated dimethyl sulfoxide (DMSO-*d*₆), deuterated chloroform (CDCl₃), and deuterated methanol (CD₃OD). The internal standard was tetramethylsilane (TMS).

MS was measured using an Agilent 1200/1290 DAD-6110/6120 Quadrupole MS liquid spectrometer (manufacturer: Agilent, MS model: 6110/6120 Quadrupole MS). waters ACQuity UPLC-QD/SQD (manufacturer: waters, MS Model: waters ACQuity Qda Detector/waters SQ Detector) THERMO Ultimate 3000- Q Exactive (manufacturer: THERMO, MS Model: THERMO Q 15 Exactive).

HPLC analysis was performed using an Agilent 1260DAD high-pressure liquid chromatograph (Sunfire C18 150×4.6 mm column) and a Thermo U3000 high-pressure liquid chromatograph (Gimini C18 150×4.6 mm column).

XRPD stands for X-ray powder diffraction detection: the measurement was carried out using a BRUKER D8 X-ray diffractometer, with specific acquisition information: Cu anode (40 kV, 40 mA), radiation: monochromatic Cu-Ka radiation (1=1.5418 Å). Scanning mode: *θ*/2*θ*, scanning range: 3-48°.

DSC stands for differential scanning calorimetry: the measurement was performed using a METTLER TOLEDO DSC 3+ differential scanning calorimeter with a heating rate of 10°C/min, from 25 to 300°C or from 25 to 350°C, and a nitrogen purge rate of 50 mL/min.

TGA stands for thermogravimetric analysis: the test was performed using a METTLER TOLEDO TGA 2 thermogravimetric analyzer with a heating rate of 10°C/min. For the specific temperature range, refer to the corresponding pattern, and the nitrogen purge rate was 50 mL/min.

DVS stands for dynamic moisture sorption: using the Surface Measurement Systems instrument, humidity started at 50% and the humidity range investigated was 0%-95% with a step of 10%. The judgment standard is that the mass change of each gradient dM/dT is ≤ 0.002%, TMAX is 360 min, and there are two cycles.

The known starting materials disclosed herein can be synthesized by methods known in the art or purchased from ABCR GmbH & Co. KG, Acros Organics, Aldrich Chemical Company, Accela ChemBio Inc, Darui Chemicals, and other companies.

The reaction progress in the examples was monitored by thin layer chromatography (TLC). The developing solvent used in the reaction, the eluent system for column chromatography used to purify the compound, and the developing solvent system for thin layer chromatography include: A: dichloromethane/methanol system, B: n-hexane/ethyl acetate system. The volume ratio of the solvent was adjusted according to the polarity of the compound, and a small amount of alkaline or acidic reagents such as triethylamine and acetic acid could also be added for adjustment.

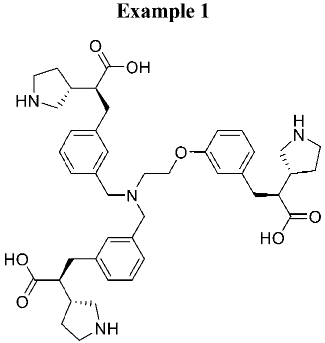

(2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid tetrahydrochloride

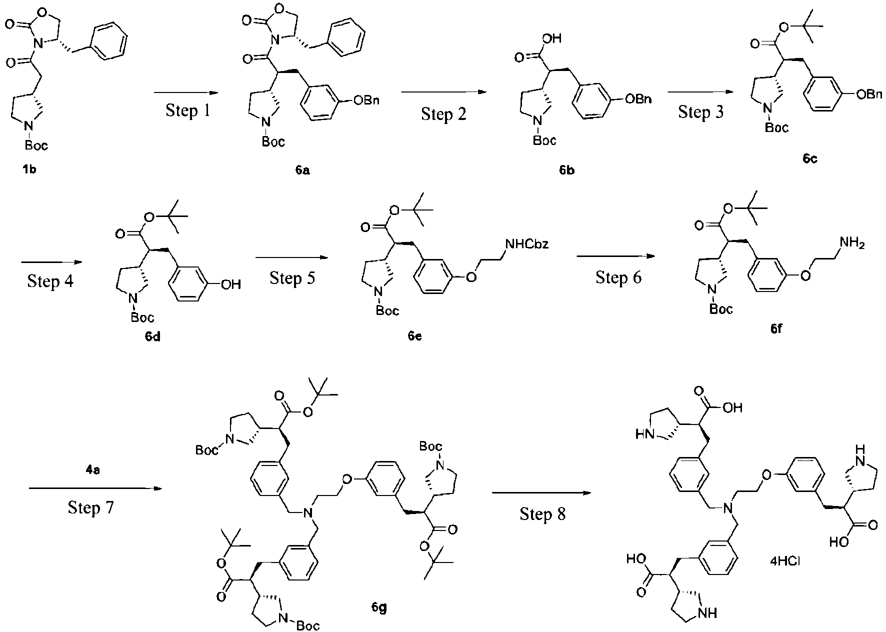

### Step 1: Preparation of tert-butyl (R)-3-((S)-1-((S)-4-benzyl-2-oxooxazolidin-3-yl)-3-(3-(benzyloxy)phenyl)-1-oxopropan-2-yl)pyrrolidine-1-carboxylate (6a)

Under nitrogen atmosphere, 1b (10.0 g, 25.74 mmol) was dissolved in tetrahydrofuran (10 mL), cooled to -30°C, and 1 M lithium bis(trimethylsilyl)amide solution in tetrahydrofuran (31 mL, 31 mmol) was added dropwise. Thereafter, the mixture was allowed to react for 30 min. A solution of 3-benzyloxybenzyl bromide (8.56 g, 30.89 mmol) in tetrahydrofuran (15 mL) was added dropwise. The reaction was continued for 1 h, and the mixture was heated to 0°C-5°C and allowed to react overnight. Saturated ammonium chloride solution (50 mL) was added, the system was extracted with methyl tert-butyl ether (50 mL × 2), washed with saturated sodium chloride solution (70 mL), dried over anhydrous sodium sulfate, filtered, and concentrated under reduced pressure. The resulting residue was purified by column chromatography (petroleum ether:ethyl acetate = 4:1) to give the title product **6a** (7.08 g, yield: 47.0%).

### Step 2: Preparation of (S)-3-(3-(benzyloxy)phenyl)-2-((R)-1-(tert-butoxycarbonyl)pyrrolidin-3-yl)propionic acid (6b)

**6a** (7 g, 11.983 mmol) was dissolved in tetrahydrofuran (70 mL) and water (35 mL), hydrogen peroxide (30%) (4.07 g, 35.95 mmol) and lithium hydroxide monohydrate (431 mg, 17.97 mmol) were added, and the system was allowed to react at room temperature overnight. The pH of the reaction mixture was adjusted to >13 with 2 M sodium hydroxide solution, and the mixture was extracted with methyl tert-butyl ether (50 mL × 2). The pH of the aqueous phase was adjusted to <3 with 2 M hydrochloric acid solution, and the mixture was extracted with methyl tert-butyl ether (50 mL × 2). The mixture was washed with saturated sodium chloride solution (70 mL), dried over anhydrous sodium sulfate, filtered, and concentrated to dryness under reduced pressure to give 3.11 g of crude product **6b,** which was directly used for the next reaction without purification.

### Step 3: Preparation of tert-butyl (R)-3-((S)-3-(3-(benzyloxy)phenyl)-1-(tert-butoxy)-1-oxopropan-2-yl)pyrrolidine-1-carboxylate (6c)

The crude product **6b** (3.1 g, 7.29 mmol) from the previous step was dissolved in 2-methyltetrahydrofuran (60 mL), and then 2-tert-butyl-1,3-diisopropylisourea (5.84 g, 29.14 mmol) was added. The system was heated to 65°C and allowed to react overnight. The mixture was filtered, and the filtrate was concentrated to dryness under reduced pressure. The resulting residue was purified by column chromatography (petroleum ether:ethyl acetate = 5:1) to give product **6c** (2.5 g, two-step yield: 43.3%).

### Step 4: Preparation of tert-butyl (R)-3-((S)-1-(tert-butoxy)-3-(3-hydroxyphenyl)-1-oxopropan-2-yl)pyrrolidine-1-carboxylate (6d)

Under hydrogen atmosphere, **6c** (2.5 g, 5.19 mmol) was dissolved in MeOH (40 mL), and Pd/C (0.80 g) was added. The mixture was allowed to react at room temperature overnight, filtered, and concentrated under reduced pressure to give product **6d** (1.78 g, yield: 87.6%).

### Step 5: Preparation of tert-butyl (R)-3-((S)-3-(3-(2-(((benzyloxy)carbonyl)amino)ethoxy) phenyl)-1-(tert-butoxy)-1-oxopropan-2-yl)pyrrolidine-1-carboxylate (6e)

**6d** (400 mg, 0.99 mmol), benzyl N-(2-bromoethyl)carbamate (280 mg, 1.09 mmol), and cesium carbonate (803 mg, 2.47 mmol) were added to N,N-dimethylformamide (8 mL), and the mixture was heated to 80°C to react for 3 h. Water (40 mL) was added, the system was extract with ethyl acetate (30 mL × 2), washed with saturated sodium chloride solution (50 mL), dried over anhydrous sodium sulfate, filtered, concentrated under pressure to give a residue, and the residue was purified by column chromatography (petroleum ether: ethyl acetate = 8:1) to give product **6e** (300 mg, yield 53.5%).

### Step 6: Preparation of tert-butyl (R)-3-((S)-3-(3-(2-aminoethoxy)phenyl)-1-(tert-butoxy)-1-oxopropan-2-yl)pyrrolidine-1-carboxylate (6f)

Under hydrogen atmosphere, **6e** (300 mg, 0.528 mmol) was dissolved in MeOH (5 mL), Pd/C (100 mg) was added and the system was allowed to react at room temperature overnight. The mixture was filtered and concentrated under reduced pressure to give product **6f** (150 mg, yield 65.4%).

### Step 7: Preparation of tert-butyl (3R)-3-[(2S)-1-(tert-butoxy)-3-(3- {[(2- {3-[(2S)-3-(tert-butoxy)-2-[(3R)-1-[(tert-butoxy)carbonyl]pyrrolidin-3-yl]-3-oxopropyl]phenoxy}ethyl)({3-[(2S)-3-(tert-butoxy)-2-[(3R)-1-[(tert-butoxy)carbonyl]pyrrolidin-3-yl]-3-oxopropyl]phenyl}methyl) amino]methyl}phenyl)-1-oxopropan-2-yl]pyrrolidine-1-carboxylate (6g)

Under ice bath, **6f** (150 mg, 0.35 mmol) and **4a** (278.6 mg, 0.69 mmol) were dissolved in isopropanol (6 mL), 1 drop of glacial acetic acid was added, and the mixture was kept to react for 30 min. Sodium triacetoxyborohydride (292.6 mg, 1.38 mmol) was added, and the mixture was slowly warmed to room temperature and allowed to react overnight. The pH was adjusted to 8-9 with saturated sodium bicarbonate solution, the system was extracted with ethyl acetate (40 mL × 2), washed with saturated sodium chloride solution (60 mL), dried over anhydrous sodium sulfate, filtered, and concentrated to give 480 mg of a crude product. The crude product was purified by Pre-HPLC to give product **6g** (250 mg, yield 59.9%).

### Step 8: Preparation of (2S)-3-(3-{[(2-{3-[(2S)-2-carboxyl-2-[(3R)-pyrrolidin-3-yl]ethyl] phenoxy}ethyl)({3-[(2S)-2-carboxyl-2-[(3R)-pyrrolidin-3-yl]ethyl]phenyl}methyl)amino]methyl} phenyl)-2-[(3R)-pyrrolidin-3-yl]propionic acid tetrahydrochloride

**6g** (100 mg, 0.083 mmol) was added to a 4 M hydrochloric acid solution in dioxane (4 mL) and the mixture was stirred at room temperature overnight. The supernatant was decanted to give a solid, which was washed with ethyl acetate and lyophilized to give the title product (50 mg, yield 68.5%). X-ray powder diffraction analysis showed that the X-ray powder diffraction pattern had no obvious characteristic peaks, as shown in Fig. 18, and it was defined as amorphous.
MS m/z (ESI): 741.3[M+H]⁺.

¹H NMR (400 MHz, D₂O): 6 7.42-7.16 (m, 9H), 6.86 (d, *J= 7.6* Hz, 1H), 6.70 (dd, *J =* 8.3, 2.6 Hz, 1H), 6.65 (s, 1H), 4.43 (s, 4H), 4.14-3.97 (m, 2H), 3.62-3.45 (m, 5H), 3.45-3.30 (m, 3H), 3.29-3.14 (m, 3H), 3.07-2.98 (m, 3H), 2.93-2.63 (m, 9H), 2.61-2.39 (m, 3H), 2.24-2.02 (m, 3H), 1.78-1.64 (m, 3H).

1g of the hydrochloride salt prepared according to the above example was taken, an aqueous sodium bicarbonate solution (4 eq., 5.4 mmol, about 0.45g of sodium bicarbonate) was added, and the mixture was stirred for 2-4 h. The system was passed through a reversed phase column C18 (with an elution gradient of acetonitrile/water of 1:9 to 1:1), freeze-dried to give (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid (compound A). X-ray powder diffraction analysis showed that the X-ray powder diffraction pattern had no obvious characteristic peaks, as shown in Fig. 1, and it was defined as amorphous.

Preparation of intermediate **1b:** Preparation of tert-butyl (R)-3-(2-((S)-4-benzyl-2-oxooxazolidin-3-yl)-2-oxoethyl)pyrrolidine-1-carboxylate **(1b)**

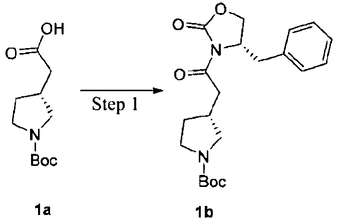

The raw material (R)-N-tert-butyloxycarbonyl-3-tetrahydropyrrolylacetic acid **la** (9 g, 39 mmol) was dissolved in anhydrous tetrahydrofuran (100 mL), cooled to 0°C, and triethylamine (13.6 mL, 98 mmol) was added. The mixture was stirred to react at 0°C for 5 min. Pivaloyl chloride (5.8 g, 47.1 mmol) was added at a temperature not exceeding 10°C. The mixture was stirred to react for 15 min, then lithium chloride (1.9 g, 47.1 mmol) and a tetrahydrofuran solution (100 mL) containing (S)-4-benzyl-2-oxazolidinone (6.9 g, 39 mmol) were added. The reaction mixture was warmed to room temperature and stirred for 24 h. 2M aqueous hydrochloric acid solution (500 mL) was added, and the organic phase was separated and concentrated. The residue was purified by silica gel flash column chromatography (petroleum ether/ethyl acetate) to give oily liquid **1b** (12.5 g, yield 82%).
MS (ESI): m/z = 411.1 [M+Na]⁺.

Preparation of intermediate **4a:** tert-butyl (R)-3-((S)-1-(tert-butoxy)-3-(3-formylphenyl)-1-oxopropan-2-yl)pyrrolidine-1-carboxylate **(4a)**

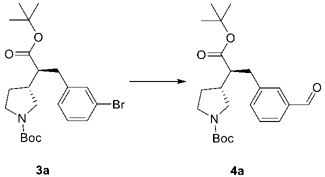

3a prepared according to Preparation 16 in patent WO2020247429, N-formyl saccharin (428 mg, 2.0 mmol), palladium acetate (40 mg, 0.2 mmol), 1,4-bis(diphenylphosphino)butane (83 mg, 0.2 mmol), and triethylsilane (0.24 mL, 1.5 mmol) were added to a microwave reaction tube, and then N,N-dimethylformamide (6 mL) was added. After nitrogen replacement, the reaction was carried out at 75°C overnight. After the reaction was completed as monitored by LCMS, the reaction mixture was cooled to room temperature and filtered by centrifugation. The filtrate was purified by reverse phase flash column chromatography (acetonitrile/water) to give compound **4a** (270 mg, yield 76%) as a yellow oil.
MS (ESI): m/z = 404.5 [M+H]⁺.

### Test Example 1: Test on activity of compounds in inhibiting Lp(a) assembly

The present disclosure utilizes a dual-antibody ELISA method to detect the assembly efficiency of Apo(a) and ApoB proteins. The antibodies are ApoB-Capture antibody (Mabtech) and Apo(a)-Detector antibody (Abcam). The test samples are plasma from human transgenic hApo(a) and hApoB mice, respectively, with 500-fold dilution when used.

Experimental steps: Equal amounts of Apo(a) and ApoB sera were respectively mixed with the test compound subjected to gradient concentration dilution (maximum concentration 100 nM, 3-fold gradient dilution), and the mixture was incubated in a 37°C incubator for 2 h. Then, EACA was added at a final concentration of 150 mM to terminate the reaction. The reaction mixture was added to an ELISA test plate pre-coated with ApoB-Capture antibody, incubated at room temperature for 2 h, and then the plate was washed 4 times with the wash buffer; biotin-labeled Apo(a)-Detector antibody was added, the system was incubated at room temperature for 1 h, the plate was washed and the colorimetric substrate TMB solution was added; after incubation at room temperature for 15 min, a reaction stop solution was added, the system was mixed, and the absorbance at 450 nm was immediately detected using a microplate reader. Finally, data analysis and IC₅₀ calculation were carried out using GraphPad Prism 9 software.

The 0% inhibition of the assembly of Apo(a) and ApoB proteins corresponds to the OD value when the compound concentration is 0 (1% DMSO); the 100% inhibition of the assembly of Apo(a) and ApoB proteins corresponds to the OD value when only ApoB protein solution (diluted hApoB mouse plasma) is added. IC₅₀ = 0.41 nM.

### Test Example 2: In vivo pharmacokinetic study in beagle dogs

With beagle dogs as test animals, the drug concentration in the plasma of beagle dogs at different time points after oral administration of the compound of the present disclosure was determined by using LC/MS/MS. The pharmacokinetic behavior of the compound of the present disclosure in beagle dogs was studied to evaluate its pharmacokinetic characteristics.

### Experimental animals: 2 healthy male beagle dogs aged 8 to 36 months per group

Drug preparation: A certain amount of drug was weighed and prepared into a 2 mg/mL colorless clear solution with normal saline.

Administration: Beagle dogs were fasted overnight and administered with the drug by gavage. The dosage of reference 1 and the compound of Example 6 was both 10 mg/kg.

Procedure: Beagle dogs were administered by gavage with the compound of the present disclosure. Approximately 0.6 mL of blood was collected by peripheral venipuncture at 0.25, 0.5, 1, 2, 4, 6, 8, and 24 h after administration. The blood was placed in a tube containing EDTA-K2 and centrifuged at 2000 rpm for 10 min at approximately 4°C to separate the plasma, which was then stored at -75°C.

Determination of the content of the test compound in beagle dog plasma at different blood collection time points: 33 µL of beagle dog plasma (30 µL plasma and 3 µL blank) collected at each time point after administration was added to 18 µL of 6% perchloric acid solution, vortexed for 30 s, and centrifuged at 3900 rpm at 4°C for 15 min. Then, 200 µL of alkaline (pH 10-11 as adjusted with ammonia water) acetonitrile solution containing internal standard dexamethasone was added to precipitate the protein. The mixture was vortexed for 30 s and centrifuged at 3900 rpm at 4°C for 15 min. The supernatant of the plasma sample was taken, diluted 3-fold with water, and 8 µL was taken for LC/MS/MS analysis.

### Pharmacokinetic parameter results

After the blood drug concentration was determined by LC/MS/MS analysis, the pharmacokinetic parameters were calculated using WinNonlin 6.1 software and the non-compartmental model method. The pharmacokinetic parameters of the compounds of the present disclosure in beagle dogs are shown in Table 1 below.

**Table 1 Pharmacokinetic parameters of the compounds of the present disclosure in beagle dogs**

| No. | Dosage | Maximum blood concentration | Area under curve | Half life |
|---|---|---|---|---|
| | mg/kg | Cmax (ng/mL) | AUC (ng/mL*h) | T_{1/2} (h) |
| Reference 1 | 10 | 1628 | 22950 | 20.7 |
| Compound A hydrochloride | 10 | 2473 | 28594 | 18.3 |

Conclusion: The compounds of the present disclosure have better oral absorption, better systemic exposure, longer half-life, so that they have excellent pharmacokinetic characteristics and have obvious advantages when administered orally.

Reference 1 is the compound of Example 1 in patent CN114008021, which is obtained according to the preparation method of Example 1 in patent CN114008021.

### Example 2 Preparation of crystal form A of compound A

5 mg of compound A (prepared according to the method of Example 1) was taken and placed under 93% RH conditions. The product was identified as crystal form A by X-ray powder diffraction analysis, with its XRPD pattern shown in Fig. 2 and the positions of its characteristic peaks shown in Table 2.

**Table 2**

| Peak No. | 20 [° or degree] | d[Å] | Relative intensity % |
|---|---|---|---|
| 1 | 8.343 | 10.58956 | 100.0 |
| 2 | 12.559 | 7.04245 | 35.9 |
| 3 | 15.612 | 5.67143 | 59.4 |
| 4 | 18.020 | 4.91867 | 62.3 |
| 5 | 21.028 | 4.22142 | 78.7 |

### Example 3 Preparation of crystal form B of compound A

100 mg of compound A (prepared according to the method in Example 1) was taken, 1.0 mL of water was added, and the mixture was pulped at room temperature and centrifuged to give the product.

The product was identified as crystal form B by X-ray powder diffraction analysis, with its XRPD pattern shown in Fig. 3 and the positions of its characteristic peaks shown in Table 3.

**Table 3**

| Peak No. | 20 [° or degree] | d[Å] | Relative intensity % |
|---|---|---|---|
| 1 | 7.794 | 11.33436 | 100.0 |
| 2 | 11.832 | 7.47343 | 10.2 |
| 3 | 12.642 | 6.99661 | 8.6 |
| 4 | 15.530 | 5.70108 | 17.3 |
| 5 | 19.456 | 4.55876 | 20.9 |
| 6 | 20.559 | 4.31652 | 35.8 |
| 7 | 21.500 | 4.12970 | 14.1 |
| 8 | 22.264 | 3.98982 | 9.7 |
| 9 | 26.589 | 3.34979 | 5.3 |
| 10 | 29.097 | 3.06652 | 11.1 |

### Example 4 Preparation of crystal form C of compound A

100 mg of compound A (prepared according to the method in Example 1) was taken, 1.0 mL of water was added, and the mixture was pulped at room temperature and centrifuged, the solid was dried to give the product.

The product was identified as crystal form C by X-ray powder diffraction analysis, with its XRPD pattern shown in Fig. 4 and the positions of its characteristic peaks shown in Table 4.

**Table 4**

| Peak No. | 20 [° or degree] | d[Å] | Relative intensity % |
|---|---|---|---|
| 1 | 8.547 | 10.33686 | 85.7 |
| 2 | 8.847 | 9.98752 | 100.0 |
| 3 | 10.487 | 8.42855 | 14.0 |
| 4 | 13.567 | 6.52125 | 71.3 |
| 5 | 15.576 | 5.68459 | 35.4 |
| 6 | 19.169 | 4.62634 | 72.7 |
| 7 | 21.973 | 4.04195 | 12.6 |
| 8 | 27.025 | 3.29671 | 4.4 |

### Example 5 Preparation of crystal form C of compound A

10 mg of compound A (prepared according to the method in Example 1) was taken, 0.1 mL of the solvent as shown in Table 5 was added, and the mixture was pulped at room temperature and centrifuged, the solid was dried to give the product.

**Table 5**

| NO. | Solvent | Crystal form |
|---|---|---|
| 1 | Isopropanol | Crystal form C |
| 2 | Ethanol | Crystal form C |
| 3 | Acetone | Crystal form C |

### Example 6 Preparation of crystal form C of compound A

5 mg of compound A (prepared according to the method in Example 1) was taken, 0.3 mL of tetrahydrofuran/ethyl acetate (v/v = 2:1) was added, 1.2 mL of water was added, and the mixture was pulped at room temperature and centrifuged, the solid was dried to give the product.

### Example 7 Preparation of crystal form D of compound A

50 mg of compound A (prepared according to the method in Example 1) was taken, 0.5 mL of 10% water/tetrahydrofuran was added, and the mixture was pulped at room temperature and centrifuged, the solid was dried to give the product.

The product was identified as free crystal form D by X-ray powder diffraction analysis, with its XRPD pattern shown in Fig. 5 and the positions of its characteristic peaks shown in Table 6.

The DSC pattern showed that the endothermic peaks were 65.77 and 256.41°C. TGA showed a weight loss of 9.9% in the range of 31-221°C.

**Table 6**

| Peak No. | 20 [° or degree] | d[Å] | Relative intensity % |
|---|---|---|---|
| 1 | 7.928 | 11.14316 | 25.7 |
| 2 | 13.834 | 6.39638 | 69.1 |
| 3 | 16.316 | 5.42826 | 71.6 |
| 4 | 20.830 | 4.26102 | 100.0 |

### Example 8: Preparation of potassium salt crystal form α

10 mg of compound A was added to 0.2 mL of ethanol, and then 28 µL of 2M aqueous potassium hydroxide solution was added. The mixture was pulped at room temperature, centrifuged, and the solid was dried to give the product.

The product was identified as potassium salt crystal form α by X-ray powder diffraction analysis, with its XRPD pattern shown in Fig. 6 and the positions of its characteristic peaks shown in Table 7.

**Table 7**

| Peak No. | 20 [° or degree] | d[Å] | Relative intensity % |
|---|---|---|---|
| 1 | 5.544 | 15.92719 | 42.50 |
| 2 | 8.016 | 11.02096 | 100.00 |
| 3 | 14.376 | 6.15604 | 17.30 |
| 4 | 19.356 | 4.58212 | 55.20 |
| 5 | 21.537 | 4.12282 | 42.20 |

### Example 9: Preparation of arginine salt crystal form a

5 mg of compound A was added to 0.1 mL of 10% water/tetrahydrofuran, and then 14 µL of 1M aqueous arginine solution was added. The mixture was pulped at room temperature, centrifuged, and the solid was dried to give the product.

The product was identified as arginine salt crystal form a by X-ray powder diffraction analysis, with its XRPD pattern shown in Fig. 7 and the positions of its characteristic peaks shown in Table 8.

**Table 8**

| Peak No. | 20 [° or degree] | d[Å] | Relative intensity % |
|---|---|---|---|
| 1 | 8.191 | 10.78557 | 79.60 |
| 2 | 10.598 | 8.34084 | 38.90 |
| 3 | 15.844 | 5.58906 | 47.60 |
| 4 | 18.447 | 4.80573 | 54.00 |
| 5 | 20.119 | 4.40998 | 100.00 |
| 6 | 23.536 | 3.77696 | 6.80 |
| 7 | 24.226 | 3.67084 | 26.40 |
| 8 | 28.333 | 3.14737 | 10.40 |

### Example 10: Preparation of sodium salt crystal form I

10 mg of compound A was added to 0.2 mL of 10% water/tetrahydrofuran, and then 8 µL of 2M aqueous sodium hydroxide solution was added. The mixture was pulped at room temperature, centrifuged, and the solid was dried to give the product.

The product was identified as sodium salt crystal form I by X-ray powder diffraction analysis, with its XRPD pattern shown in Fig. 8 and the positions of its characteristic peaks shown in Table 9.

**Table 9**

| Peak No. | 20 [° or degree] | d[Å] | Relative intensity % |
|---|---|---|---|
| 1 | 7.834 | 11.27621 | 21.90 |
| 2 | 13.831 | 6.39745 | 36.10 |
| 3 | 16.194 | 5.46905 | 21.60 |
| 4 | 18.556 | 4.77773 | 100.00 |

### Example 11: Preparation of meglumine salt crystal form a

10 mg of compound A was added to 0.2 mL of ethanol, and then 57 µL of 1M aqueous meglumine solution was added. The mixture was pulped at room temperature, centrifuged, and the solid was dried to give the product.

The product was identified as meglumine salt crystal form a by X-ray powder diffraction analysis, with its XRPD pattern shown in Fig. 9 and the positions of its characteristic peaks shown in Table 10.

**Table 10**

| Peak No. | 20 [° or degree] | d[Å] | Relative intensity % |
|---|---|---|---|
| 1 | 5.361 | 16.47269 | 100.00 |
| 2 | 5.879 | 15.02022 | 36.40 |
| 3 | 7.118 | 12.40899 | 57.90 |
| 4 | 8.069 | 10.94802 | 89.50 |
| 5 | 8.827 | 10.00990 | 75.40 |
| 6 | 9.748 | 9.06585 | 14.60 |
| 7 | 12.268 | 7.20870 | 7.30 |
| 8 | 13.795 | 6.41422 | 9.80 |
| 9 | 16.230 | 5.45688 | 15.90 |
| 10 | 17.866 | 4.96082 | 4.90 |
| 11 | 19.069 | 4.65035 | 50.10 |
| 12 | 21.864 | 4.06187 | 26.70 |
| 13 | 26.223 | 3.39564 | 15.40 |

### Example 12: Preparation of sodium salt amorphous form

5 mg of compound A was added to 0.1 mL of ethyl acetate, and then 7 µL of 2M aqueous sodium hydroxide solution was added. The mixture was pulped at room temperature, centrifuged, and the solid was dried to give the product.

As identified by X-ray powder diffraction analysis, the XRPD pattern had no obvious characteristic peaks, and the product is sodium salt amorphous form.

### Example 13: Preparation of arginine salt amorphous form

5 mg of compound A was added to 0.1 mL of ethyl acetate, and then 14 µL of 1M aqueous arginine solution was added. The mixture was pulped at room temperature, centrifuged, and the solid was dried to give the product.

As identified by X-ray powder diffraction analysis, the XRPD pattern had no obvious characteristic peaks, and the product is arginine salt amorphous form.

### Example 14: Preparation of meglumine salt amorphous form

5 mg of compound A was added to 0.1 mL of ethanol, and then 14 µL of 1M aqueous meglumine solution was added. The mixture was pulped at room temperature, centrifuged, and the solid was dried to give the product.

As identified by X-ray powder diffraction analysis, the XRPD pattern had no obvious characteristic peaks, and the product is meglumine salt amorphous form.

### Example 15: Preparation of maleate crystal form a

10 mg of compound A was added to 0.2 mL of 10% water/tetrahydrofuran, and then 14 µL of 1M aqueous maleic acid solution was added. The mixture was pulped at room temperature, centrifuged, and the solid was dried to give the product.

The product was identified as maleate crystal form a by X-ray powder diffraction analysis, with its XRPD pattern shown in Fig. 10 and the positions of its characteristic peaks shown in Table 11.

**Table 11**

| Peak No. | 20 [° or degree] | d[Å] | Relative intensity % |
|---|---|---|---|
| 1 | 7.907 | 11.17270 | 37.30 |
| 2 | 15.612 | 5.67143 | 68.30 |
| 3 | 19.174 | 4.62514 | 67.60 |
| 4 | 20.701 | 4.28738 | 100.00 |

### Example 16: Preparation of hippurate crystal form α

5 mg of compound A was added to 0.1 mL of ethyl acetate, and then 14 µL of 1M aqueous hippuric acid solution was added. The mixture was pulped at room temperature and centrifuged to give the product.

The product was identified as hippurate crystal form α by X-ray powder diffraction analysis, with its XRPD pattern shown in Fig. 11 and the positions of its characteristic peaks shown in Table 12.

**Table 12**

| Peak No. | 20 [° or degree] | d[Å] | Relative intensity % |
|---|---|---|---|
| 1 | 4.791 | 18.43054 | 16.50 |
| 2 | 5.551 | 15.90653 | 33.40 |
| 3 | 9.410 | 9.39116 | 63.30 |
| 4 | 11.209 | 7.88730 | 35.00 |
| 5 | 11.663 | 7.58175 | 18.70 |
| 6 | 13.522 | 6.54303 | 100.00 |
| 7 | 14.175 | 6.24301 | 37.30 |
| 8 | 14.534 | 6.08960 | 38.60 |
| 9 | 15.249 | 5.80579 | 4.50 |
| 10 | 16.709 | 5.30164 | 29.20 |
| 11 | 18.118 | 4.89240 | 22.50 |
| 12 | 19.956 | 4.44563 | 58.60 |
| 13 | 21.022 | 4.22253 | 38.30 |
| 14 | 21.337 | 4.16091 | 75.30 |
| 15 | 22.945 | 3.87286 | 80.50 |
| 16 | 23.834 | 3.73037 | 24.90 |
| 17 | 25.062 | 3.55026 | 9.80 |
| 18 | 25.736 | 3.45876 | 13.50 |
| 19 | 26.770 | 3.32746 | 5.90 |
| 20 | 27.461 | 3.24533 | 9.30 |
| 21 | 29.280 | 3.04769 | 11.80 |
| 22 | 31.013 | 2.88124 | 13.90 |
| 23 | 35.166 | 2.54989 | 5.30 |

### Example 17: Preparation of hippurate crystal form β

5 mg of compound A was added to 0.1 mL of ethyl acetate, and then 14 µL of 1M aqueous hippuric acid solution was added. The mixture was pulped at room temperature, centrifuged, and the solid was dried to give the product.

The product was identified as hippurate crystal form β by X-ray powder diffraction analysis, with its XRPD pattern shown in Fig. 12 and the positions of its characteristic peaks shown in Table 13.

**Table 13**

| Peak No. | 20 [° or degree] | d[Å] | Relative intensity % |
|---|---|---|---|
| 1 | 4.944 | 17.85824 | 38.60 |
| 2 | 9.978 | 8.85719 | 5.40 |
| 3 | 14.737 | 6.00603 | 100.00 |
| 4 | 17.143 | 5.16830 | 14.90 |
| 5 | 20.109 | 4.41218 | 61.70 |
| 6 | 24.335 | 3.65464 | 2.20 |
| 7 | 25.971 | 3.42807 | 3.80 |

### Example 18: Preparation of sulfate amorphous form

5 mg of compound A was added to 0.1 mL of 10% water/tetrahydrofuran, and then 8 µL of 1.8M aqueous sulfuric acid solution was added. The mixture was pulped at room temperature, centrifuged, and the solid was dried to give the product.

As identified by X-ray powder diffraction analysis, the XRPD pattern had no characteristic peaks, and the product is sulfate amorphous form.

### Example 19: Preparation of phosphate amorphous form

5 mg of compound A was added to 0.1 mL of ethanol, and then 10 µL of 1.5M aqueous phosphoric acid solution was added. The mixture was pulped at room temperature, centrifuged, and the solid was dried to give the product.

As identified by X-ray powder diffraction analysis, the XRPD pattern had no characteristic peaks, and the product is phosphate amorphous form.

### Example 20: Preparation of methanesulfonate amorphous form

5 mg of compound A was added to 0.1 mL of ethanol, and then 9 µL of 1.5M aqueous methanesulfonic acid solution was added. The mixture was pulped at room temperature, centrifuged, and the solid was dried to give the product.

As identified by X-ray powder diffraction analysis, the XRPD pattern had no characteristic peaks, and the product is methanesulfonate amorphous form.

### Example 21: Preparation of maleate amorphous form

5 mg of compound A was added to 0.1 mL of ethanol, and then 14 µL of 1M aqueous maleic acid solution was added. The mixture was pulped at room temperature, centrifuged, and the solid was dried to give the product.

As identified by X-ray powder diffraction analysis, the XRPD pattern had no characteristic peaks, and the product is maleate amorphous form.

### Example 22: Preparation of fumarate amorphous form

5 mg of compound A was added to 0.1 mL of ethanol, and then 21 µL of 0.7M fumaric acid solution in methanol was added. The mixture was pulped at room temperature, centrifuged, and the solid was dried to give the product.

As identified by X-ray powder diffraction analysis, the XRPD pattern had no characteristic peaks, and the product is fumarate amorphous form.

### Example 23: Preparation of citrate amorphous form

5 mg of compound A was added to 0.1 mL of ethanol, and then 14 µL of 0.7M aqueous citric acid solution was added. The mixture was pulped at room temperature, centrifuged, and the solid was dried to give the product.

As identified by X-ray powder diffraction analysis, the XRPD pattern had no characteristic peaks, and the product is citrate amorphous form.

### Example 24: Preparation of hydochloride (1:1) crystal form a

10 mg of compound A was added to 0.07 mL of methanol, and then 9 µL of 5M aqueous hydrochloric acid solution was added. The mixture was pulped at room temperature, centrifuged, and the solid was dried to give the product.

The product was identified as hydrochloride crystal form a by X-ray powder diffraction analysis, with its XRPD pattern shown in Fig. 13 and the positions of its characteristic peaks shown in Table 14.

The DSC pattern showed that the endothermic peaks were 59.78 and 285.05°C. TGA showed a weight loss of 4.4% in the range of 32-160°C. Ion chromatography detection showed that the chloride ion content was 4.75%. The DVS hygroscopicity test results showed that the weight gain was 30% at 80% RH.

**Table 14**

| Peak No. | 20 [° or degree] | d[Å] | Relative intensity % |
|---|---|---|---|
| 1 | 6.385 | 13.83068 | 100.0 |
| 2 | 8.530 | 10.35819 | 59.4 |
| 3 | 13.533 | 6.53794 | 81.6 |
| 4 | 16.203 | 5.46582 | 93.4 |
| 5 | 21.244 | 4.17895 | 86.6 |

### Example 25: Preparation of hydochloride (1:1) crystal form a

10 mg of compound A was added to 0.07 mL of ethyl acetate, and then 9 µL of 5M aqueous hydrochloric acid solution was added. The mixture was pulped at room temperature, centrifuged, and the solid was dried to give the target product.

### Example 26: Preparation of hydochloride (1:1) crystal form a

10 mg of compound A was added to 0.07 mL of 1,4-dioxane, and then 9 µL of 5M aqueous hydrochloric acid solution was added. The mixture was pulped at room temperature, centrifuged, and the solid was dried to give the target product.

### Example 27: Preparation of hydochloride crystal form b

10 mg of compound A was added to 0.07 mL of acetonitrile, and then 9 µL of 5M aqueous hydrochloric acid solution was added. The mixture was pulped at room temperature and centrifuged to give the product.

The product was identified as hydrochloride crystal form b by X-ray powder diffraction analysis, with its XRPD pattern shown in Fig. 14 and the positions of its characteristic peaks shown in Table 15.

**Table 15**

| Peak No. | 20 [° or degree] | d[Å] | Relative intensity % |
|---|---|---|---|
| 1 | 8.059 | 10.96239 | 57.2 |
| 2 | 10.359 | 8.53307 | 24.3 |
| 3 | 14.666 | 6.03506 | 23.4 |
| 4 | 16.017 | 5.52904 | 32.9 |
| 5 | 17.295 | 5.12333 | 29.0 |
| 6 | 17.806 | 4.97742 | 38.3 |
| 7 | 19.412 | 4.56903 | 73.1 |
| 8 | 20.726 | 4.28219 | 80.3 |
| 9 | 21.843 | 4.06577 | 100.0 |
| 10 | 24.158 | 3.68113 | 23.2 |

### Example 28: Preparation of hydochloride (1:1) crystal form c

10 mg of compound A was added to 0.07 mL of 10% water/tetrahydrofuran (v/v), and then 9 µL of 5M aqueous hydrochloric acid solution was added. The mixture was pulped at room temperature and centrifuged to give the product.

The product was identified as hydrochloride crystal form c by X-ray powder diffraction analysis, with its XRPD pattern shown in Fig. 15 and the positions of its characteristic peaks shown in Table 16.

The DSC pattern showed that the endothermic peaks were 70.77, 114.11, 147.43, 227.41, 282.72°C. TGA showed a weight loss of 3.8% in the range of 32-122°C and a weight loss of 4.2% in the range of 122-225°C.

**Table 16**

| Peak No. | 20 [° or degree] | d[Å] | Relative intensity % |
|---|---|---|---|
| 1 | 9.395 | 9.40614 | 94.4 |
| 2 | 11.238 | 7.86719 | 43.5 |
| 3 | 13.495 | 6.55608 | 100.0 |
| 4 | 20.153 | 4.40262 | 15.7 |
| 5 | 21.169 | 4.19363 | 7.3 |
| 6 | 23.125 | 3.84314 | 15.4 |

### Example 29: Preparation of hydochloride (1:1) crystal form d

10 mg of compound A was added to 0.07 mL of 10% water/isopropanol, and then 9 µL of 5M aqueous hydrochloric acid solution was added. The mixture was pulped at room temperature and centrifuged to give the product.

The product was identified as hydrochloride crystal form d by X-ray powder diffraction analysis, with its XRPD pattern shown in Fig. 16 and the positions of its characteristic peaks shown in Table 17.

The DSC pattern showed that the endothermic peaks were 97.05, 117.48, 142.73, 284.01°C. TGA showed a weight loss of 6.3% in the range of 33-138°C.

**Table 17**

| Peak No. | 20 [° or degree] | d[Å] | Relative intensity % |
|---|---|---|---|
| 1 | 8.128 | 10.86874 | 66.6 |
| 2 | 12.216 | 7.23944 | 34.5 |
| 3 | 12.995 | 6.80723 | 37.3 |
| 4 | 13.683 | 6.46638 | 16.8 |
| 5 | 14.962 | 5.91638 | 36.4 |
| 6 | 16.354 | 5.41598 | 35.6 |
| 7 | 17.370 | 5.10140 | 22.3 |
| 8 | 18.272 | 4.85134 | 15.4 |
| 9 | 19.268 | 4.60292 | 51.0 |
| 10 | 19.965 | 4.44367 | 31.1 |
| 11 | 20.401 | 4.34972 | 63.1 |
| 12 | 21.319 | 4.16437 | 30.3 |
| 13 | 23.032 | 3.85840 | 100.0 |
| 14 | 24.103 | 3.68936 | 6.1 |
| 15 | 24.742 | 3.59554 | 40.7 |
| 16 | 26.134 | 3.40703 | 17.7 |
| 17 | 26.849 | 3.31793 | 8.5 |
| 18 | 28.053 | 3.17824 | 28.1 |
| 19 | 29.933 | 2.98268 | 10.4 |

### Example 30: Preparation of hydochloride (1:1) crystal form e

100 mg of compound A was added to 0.6 mL of 10% water/isopropanol, and then 27 µL of 5M aqueous hydrochloric acid solution was added. The mixture was pulped at room temperature, centrifuged, and the solid was dried to give the product.

The product was identified as hydrochloride crystal form e by X-ray powder diffraction analysis, with its XRPD pattern shown in Fig. 17 and the positions of its characteristic peaks shown in Table 18.

The DSC pattern showed that the endothermic peaks were 76.58, 108.43, 146.76, 287.37°C. TGA showed a weight loss of 6.1% in the range of 30-161°C.

Ion chromatography detection showed that the chloride ion content was 5.20%. The DVS hygroscopicity test results showed that the weight gain was 18% at 80% RH.

**Table 18**

| Peak No. | 20 [° or degree] | d[Å] | Relative intensity % |
|---|---|---|---|
| 1 | 9.385 | 9.41549 | 60.0 |
| 2 | 10.651 | 8.29936 | 20.0 |
| 3 | 12.305 | 7.18715 | 23.4 |
| 4 | 14.145 | 6.25639 | 84.1 |
| 5 | 15.451 | 5.73022 | 29.2 |
| 6 | 17.783 | 4.98362 | 14.5 |
| 7 | 19.094 | 4.64431 | 31.6 |
| 8 | 19.709 | 4.50077 | 62.2 |
| 9 | 20.277 | 4.37597 | 84.5 |
| 10 | 21.583 | 4.11415 | 100.0 |
| 11 | 23.187 | 3.83290 | 31.8 |
| 12 | 23.684 | 3.75368 | 36.8 |
| 13 | 24.366 | 3.65008 | 37.7 |
| 14 | 25.156 | 3.53723 | 12.3 |
| 15 | 25.908 | 3.43619 | 21.5 |
| 16 | 29.332 | 3.04250 | 13.8 |

### Example 31: Preparation of hydochloride crystal form e

10 mg of compound A was added to 0.6 mL of acetonitrile, and then 2.7 µL of 5M aqueous hydrochloric acid solution was added. The mixture was pulped at room temperature, centrifuged, and the solid was dried to give the target product.

### Example 32: Preparation of hydochloride crystal form e

100 mg of compound A was added to 0.6 mL of 10% water/tetrahydrofuran (v/v), and then 27 µL of 5M aqueous hydrochloric acid solution was added. The mixture was pulped at room temperature, centrifuged, and the solid was dried to give the target product.

### Example 33: Preparation of hydochloride crystal form e

2 g of compound A was added to 6.0 mL of water and 10.0 mL of methanol. Then, 540 µL of 5M aqueous hydrochloric acid solution was added. The resulting solution was added to 20.0 mL of isopropanol. The mixture was pulped at room temperature, centrifuged, and the solid was dried to give the target product.

### Example 34: Preparation of hydochloride (1:1) amorphous form

125 mg of hydrochloride crystal form d of compound A was added to 2.5 mL of acetonitrile. The mixture was pulped at room temperature, centrifuged, and the solid was dried to give the target product. As identified by X-ray powder diffraction analysis, the product was identified as a hydrochloride amorphous form, with its XRPD pattern shown in Fig. 19.

### Example 35: Preparation of hydochloride (1:4) amorphous form

5 mg of the tetrahydrochloride sample prepared from compound A in Example 1 was added to the solvent as shown in Table 19. The mixture was pulped at room temperature. The crystallization process was observed. If any solid appeared, the system was centrifuged and dried to give the solid product. The product was subjected to X-ray powder diffraction analysis, and the relevant data are shown in Table 19.

**Table 19**

| NO. | Solvent | Crystal form |
|---|---|---|
| 1 | 0.05mL methanol | Colloid |
| 2 | 0.05mL H2O | Colloid |
| 3 | 0.05mL ethanol | Colloid |
| 4 | 0.05mL propylene glycol monomethyl ether | Colloid |
| 5 | 1.0mL isopropanol | Amorphous form ^{a} |
| 6 | 1.0mL acetone | Amorphous form |
| 7 | 1.0mL 2-butanone | Amorphous form |
| 8 | 1.0mL methyl isobutyl ketone | Amorphous form |
| 9 | 1.0mL ethyl acetate | Amorphous form |
| 10 | 1.0mL methyl tert-butyl ether | Amorphous form |
| 11 | 1.0mL 1,4-dioxane | Amorphous form |
| 12 | 1.0mL isopropyl acetate | Amorphous form |
| 13 | 1.0mL tetrahydrofuran | Amorphous form |
| 14 | 1.0mL acetonitrile | Amorphous form |
| 15 | 1.0mL dichloromethane | Amorphous form |
| 16 | 1.0mL n-heptane | Amorphous form |
| 17 | 0.05mL 10% water/methanol | Colloid |
| 18 | 0.05mL 7% water/ethanol | Colloid |
| 19 | 0.05mL 10% water/acetone | Amorphous form |
| 20 | 0.05mL 10% water/isopropanol | Amorphous form |
| 21 | 1.0mL isopropyl ether | Amorphous form |

| | | |
|---|---|---|
| Note: a The XRPD pattern of the sample is shown in Fig. 18. The DVS hygroscopicity test result indicates that the weight gain is 34% at 80% RH. | | |

### Example 35: Preparation of crystal form E

10 mg of compound A (prepared according to the method in Example 1) was taken, 0.5 ml of methanol was added, the mixture was pulped at 25°C for 4 days, the solid was centrifuged and dried to give the product. The product was identified as crystal form E by X-ray powder diffraction analysis, with its XRPD pattern shown in Fig. 20 and the positions of its characteristic peaks shown in Table 20.

The DSC pattern showed that the endothermic peak was 247.23°C. TGA showed a weight loss of 3.58% in the range of 36-90°C.

**Table 20**

| Peak No. | 20 [° or degree] | d[Å] | Relative intensity % |
|---|---|---|---|
| 1 | 4.671 | 18.90274 | 24.6 |
| 2 | 4.933 | 17.89950 | 34.6 |
| 3 | 6.545 | 13.49321 | 100.0 |
| 4 | 8.528 | 10.36009 | 17.1 |
| 5 | 9.850 | 8.97281 | 3.8 |
| 6 | 13.037 | 6.78540 | 7.9 |
| 7 | 15.017 | 5.89478 | 11.0 |
| 8 | 16.262 | 5.44628 | 23.1 |
| 9 | 17.349 | 5.10735 | 7.0 |
| 10 | 18.549 | 4.77958 | 4.8 |
| 11 | 19.974 | 4.44171 | 8.0 |
| 12 | 20.874 | 4.25220 | 10.8 |
| 13 | 22.149 | 4.01024 | 14.2 |

### Example 36: Preparation of crystal form E

10 mg of compound A (prepared according to the method in Example 1) was taken, 0.5 ml of methanol was added, the mixture was pulped at 50°C for 1 day, the solid was centrifuged and dried to give the target product.

### Example 37: Preparation of crystal form E

About 10 mg of the compound represented by formula (I) was weighed and dissolved in 0.5 mL of ethyl acetate/ethanol (v/v= 1: 1) with stirring. The reaction mixture was pulped at 25°C for 4 days. The solid was centrifuged and dried to give the target product.

### Example 38: Preparation of crystal form E

About 10 mg of the compound represented by formula (I) was weighed and dissolved in 0.5 mL of acetonitrile/methanol (v/v=1:1) with stirring. The reaction mixture was pulped at 25°C for 4 days. The solid was centrifuged and dried to give the target product.

### Example 39: Preparation of crystal form E

About 10 mg of the compound represented by formula (I) was weighed and dissolved in 0.5 mL of acetonitrile/methanol (v/v=1:1) with stirring. The reaction mixture was pulped at 50°C for 1 day. The solid was centrifuged and dried to give the target product.

### Test Example 3: Long-term accelerated test

The aforementioned compound A hydrochloride (1:1) crystal form a, compound A hydrochloride (1:1) crystal form e, compound A hydrochloride (1:4) amorphous form, and compound A hydrochloride (1:1) amorphous form were sealed in aluminum foil bags and placed under 25°C/60% RH and 40°C/75% RH conditions, respectively, to investigate their stability.

**Table 21 Long-term/accelerated stability of hydrochloride (1:1) crystal form a**

| **Conditions** | **Time** | **Purity (%)** | **Crystal form** |
|---|---|---|---|
| / | 0 day | 99.9 | Hydrochloride crystal form a |
| 25°C-60%RH | 7 days | 99.9 | Unchanged |
| | 14 days | 99.9 | Unchanged |
| | 1 month | 99.9 | Unchanged |
| | 3 months | 99.7 | Deliquescent |
| | 6 months | 98.5 | Deliquescent |
| 40°C-75%RH | 7 days | 99.8 | Unchanged |
| | 14 days | 98.8 | Unchanged |
| | 1 month | 97.7 | Deliquescent |
| | 3 months | 95.2 | Deliquescent |
| | 6 months | 94.8 | Deliquescent |

**Table 22 Long-term/accelerated stability of hydrochloride (1:1) crystal form e**

| **Conditions** | **Time** | **Purity (%)** | **Crystal form** |
|---|---|---|---|
| / | 0 day | 99.9 | Hydrochloride crystal form e |
| 25°C-60%RH | 7 days | 99.9 | Unchanged |
| | 14 days | 99.9 | Unchanged |
| | 1 month | 99.9 | Unchanged |
| | 3 months | 99.9 | Unchanged |
| | 6 months | 99.9 | Unchanged |
| 40°C-75%RH | 7 days | 99.9 | Unchanged |
| | 14 days | 99.9 | Unchanged |
| | 1 month | 99.9 | Unchanged |
| | 3 months | 99.9 | Unchanged |
| | 6 months | 99.9 | Unchanged |

**Table 23 Long-term/accelerated stability of hydrochloride (1:4) amorphous form**

| **Conditions** | **Time** | **Purity (%)** | **Crystal form** |
|---|---|---|---|
| / | 0 day | 99.8 | Amorphous form |
| 25°C-60%RH | 7 days | 99.8 | Unchanged |
| | 14 days | 99.8 | Unchanged |
| | 1 month | 99.8 | Unchanged |
| | 2 months | 99.8 | Deliquescent |
| | 3 months | 99.8 | Deliquescent |
| | 6 months | 99.8 | Deliquescent |
| 40°C-75%RH | 7 days | 99.8 | Unchanged |
| | 14 days | 99.8 | Unchanged |
| | 1 month | 99.8 | Deliquescent |
| | 2 months | 99.8 | Deliquescent |
| | 3 months | 99.8 | Deliquescent |
| | 6 months | 99.8 | Deliquescent |

**Table 24 Long-term/accelerated stability of hydrochloride (1:1) amorphous form**

| **Conditions** | **Time** | **Purity (%)** | **Crystal form** |
|---|---|---|---|
| / | 0 day | 98.8 | Amorphous form |
| 25°C-60%RH | 7 days | 98.8 | Unchanged |
| | 14 days | 98.8 | Unchanged |
| | 1 month | 98.7 | Crystal form a |
| | 2 months | 98.6 | Crystal form a |
| | 3 months | 98.2 | Crystal form a |
| 40°C-75%RH | 7 days | 98.3 | Crystal form a |
| | 14 days | 98.1 | Crystal form a |
| | 1 month | 97.6 | Crystal form a |
| | 2 months | 95.6 | Crystal form a |
| | 3 months | 95.3 | Crystal form a |

Conclusion: The long-term accelerated experiment showed that at 25°C/60%RH and 40°C/75%RH for 6 months, the physicochemical stability of the hydrochloride (1:1) crystal form e was good, while the hydrochloride (1:1) crystal form a, hydrochloride (1:1) amorphous form and hydrochloride (1:4) amorphous form were slightly less stable.

### Test Example 4: Long-term accelerated test

The free crystal form C of the compound as described above A was sealed in an aluminum foil bag (nitrogen-filled) and placed under 25°C/60% RH and 40°C/75% RH conditions, respectively, to investigate the stability.

**Table 25 Long-term/accelerated stability of free crystal form C**

| **Conditions** | **Time** | **Purity (%)** | **Crystal form** |
|---|---|---|---|
| / | 0 day | 99.8 | Crystal form C |
| 25°C-60%RH | 7 days | 99.8 | Unchanged |
| | 14 days | 99.8 | Unchanged |
| | 1 month | 99.8 | Unchanged |
| | 3 months | 99.7 | Unchanged |
| | 6 months | 99.7 | Unchanged |
| 40°C-75%RH | 7 days | 99.8 | Unchanged |
| | 14 days | 99.8 | Unchanged |
| | 1 month | 99.8 | Unchanged |
| | 3 months | 99.3 | Unchanged |
| | 6 months | 99.3 | Unchanged |

Conclusion: The long-term accelerated experiment showed that at 25°C/60%RH and 40°C/75%RH for 6 months, the physicochemical stability of the free crystal form C was good.

## Claims

1. A crystal form A of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl) amino]methyl}phenyl)-2-[(3R)-pyrrolidin-3-yl]propionic acid, **characterized by** an X-ray powder diffraction pattern represented by a diffraction angle 2*θ* having characteristic peaks at 8.343, 18.020, and 21.028, preferably having characteristic peaks at 8.343, 12.559, 15.612, 18.020, and 21.028, and more preferably an X-ray powder diffraction pattern represented by a diffraction angle 2*θ* as shown in Fig. 2.

2. A crystal form B of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl) amino]methyl}phenyl)-2-[(3R)-pyrrolidin-3-yl]propionic acid, **characterized by** an X-ray powder diffraction pattern represented by a diffraction angle 2*θ* having characteristic peaks at 7.794, 15.530, 19.456, 20.559, and 21.500, preferably having characteristic peaks at 7.794, 11.832, 15.530, 19.456, 20.559, 21.500, and 29.097, more preferably having characteristic peaks at 7.794, 11.832, 12.642, 15.530, 19.456, 20.559, 21.500, and 29.097, and most preferably an X-ray powder diffraction pattern represented by a diffraction angle 2*θ* as shown in Fig. 3.

3. A crystal form C of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl) amino]methyl}phenyl)-2-[(3R)-pyrrolidin-3-yl]propionic acid, **characterized by** an X-ray powder diffraction pattern represented by a diffraction angle 2*θ* having characteristic peaks at 8.547, 8.847, 13.567, 15.576, and 19.169, preferably having characteristic peaks at 8.547, 8.847, 10.487, 13.567, 15.576, and 19.169, more preferably having characteristic peaks at 8.547, 8.847, 10.487, 13.567, 15.576, 19.169, and 21.973, and most preferably an X-ray powder diffraction pattern represented by a diffraction angle 2*θ* as shown in Fig. 4.

4. A crystal form D of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl) amino]methyl}phenyl)-2-[(3R)-pyrrolidin-3-yl]propionic acid, **characterized by** an X-ray powder diffraction pattern represented by a diffraction angle 2*θ* having characteristic peaks at 13.834, 16.316, and 20.830, preferably having characteristic peaks at 7.928, 13.834, 16.316, and 20.830, and more preferably an X-ray powder diffraction pattern represented by a diffraction angle 2*θ* as shown in Fig. 5.

5. The crystal form according to any one of claims 1 to 4, wherein the 2*θ* has an error range of ±0.2.

6. A pharmaceutically acceptable salt of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl] phenyl}methyl)amino]methyl}phenyl)-2-[(3R)-pyrrolidin-3-yl]propionic acid, wherein the pharmaceutically acceptable salt is selected from hydrochloride, succinate, meglumine salt, maleate, hippurate, sulfate, phosphate, methanesulfonate, tartrate, fumarate, citrate, sodium salt, potassium salt and arginine salt.

7. The pharmaceutically acceptable salt according to claim 6, wherein the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid to acid molecules or base molecules is 1:0.2 to 1:5, and preferably 1:0.5, 1:1, 1:2, 1:3 or 1:4.

8. The pharmaceutically acceptable salt according to claim 6 or 7, wherein the chemical ratio of compound (2S)-3-(3-{[(2-{3-[(2S)-2-carboxyl-2-[(3R)-pyrrolidin-3-yl]ethyl]phenoxy}ethyl) ({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid to HCl is 1:1, 1:2 or 1:3.

9. A method for preparing the pharmaceutically acceptable salt according to claim 6 or 7, comprising a step of forming a salt of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid with an acid or a base.

10. The method according to claim 6, wherein the solvent used in the salt-forming reaction is selected from at least one of ethanol, ethyl acetate, tetrahydrofuran, acetonitrile, acetone, methanol, 1,4-dioxane, 2-methyltetrahydrofuran, ethylene glycol, isopropanol, and water.

11. A potassium salt crystal form **α** of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 5.544, 8.016, and 19.356, preferably having characteristic peaks at 5.544, 8.016, 19.356, and 21.537, and more preferably an X-ray powder diffraction pattern represented by a diffraction angle 2*θ* as shown in Fig. 6.

12. An arginine salt crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 8.191, 10.598, 15.844, 18.447, and 20.119, preferably having characteristic peaks at 8.191, 10.598, 15.844, 18.447, 20.119, and 24.226, and more preferably an X-ray powder diffraction pattern represented by a diffraction angle 2*θ* as shown in Fig. 7.

13. A sodium salt crystal form I of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 13.831 and 18.556, preferably having characteristic peaks at 7.834, 13.831, 16.194, and 18.556, and more preferably an X-ray powder diffraction pattern represented by a diffraction angle 2*θ* as shown in Fig. 8.

14. A meglumine salt crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 5.361, 5.879, 7.118, 8.069, 8.827, and 19.069, preferably having characteristic peaks at 5.361, 5.879, 7.118, 8.069, 8.827, 9.748, 16.230, and 19.069, more preferably having characteristic peaks at 5.361, 5.879, 7.118, 8.069, 8.827, 9.748, 13.795, 16.230, 19.069, 21.864, and 26.223, and most preferably an X-ray powder diffraction pattern represented by a diffraction angle 2*θ* as shown in Fig. 9.

15. A maleate crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 7.907, 15.612, 19.174, and 20.701, and preferably an X-ray powder diffraction pattern represented by a diffraction angle 2*θ* as shown in Fig. 10.

16. A hippurate crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 5.551, 9.410, 13.522, 14.534, 19.956, 21.337, and 22.945, preferably having characteristic peaks at 5.551, 9.410, 11.209, 13.522, 14.175, 14.534, 19.956, 21.022, 21.337, and 22.945, more preferably having characteristic peaks at 5.551, 9.410, 11.209, 13.522, 14.175, 14.534, 16.709, 19.956, 21.022, 21.337, and 22.945, and most preferably an X-ray powder diffraction pattern represented by a diffraction angle 2*θ* as shown in Fig. 11.

17. A hippurate crystal form β of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 4.944, 14.737, 17.143, and 20.109, preferably having characteristic peaks at 4.944, 9.978, 14.737, 17.143, and 20.109, and more preferably an X-ray powder diffraction pattern represented by a diffraction angle 2*θ* as shown in Fig. 12.

18. A hydrochloride crystal form a of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 6.385, 13.533, 16.203, and 21.244, preferably having characteristic peaks at 6.385, 8.530, 13.533, 16.203, and 21.244, and more preferably an X-ray powder diffraction pattern represented by a diffraction angle 2*θ* as shown in Fig. 13.

19. A hydrochloride crystal form b of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 8.059, 10.359, 19.412, 20.726, and 21.843, preferably having characteristic peaks at 8.059, 10.359, 16.017, 17.806, 19.412, 20.726, and 21.843, and more preferably an X-ray powder diffraction pattern represented by a diffraction angle 2*θ* as shown in Fig. 14.

20. A hydrochloride crystal form c of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 9.395, 11.238, 13.495, and 20.153, preferably having characteristic peaks at 9.395, 11.238, 13.495, 20.153, 21.169, and 23.125, and more preferably an X-ray powder diffraction pattern represented by a diffraction angle 2*θ* as shown in Fig. 15.

21. A hydrochloride crystal form d of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 8.128, 12.995, 14.962, 16.354, 20.401, and 23.032, preferably having characteristic peaks at 8.128, 12.216, 12.995, 14.962, 16.354, 19.268, 20.401, and 23.032, more preferably having characteristic peaks at 8.128, 12.216, 12.995, 14.962, 16.354, 19.268, 19.965, 20.401, 23.032, and 24.742, and most preferably an X-ray powder diffraction pattern represented by a diffraction angle 2*θ* as shown in Fig. 16.

22. A hydrochloride crystal form e of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl} methyl)amino]methyl}phenyl)-2-[(3R)-pyrrolidin-3-yl]propionic acid, **characterized by** an X-ray powder diffraction pattern represented by a diffraction angle 2*θ* having characteristic peaks at 9.385, 14.145, 19.709, 20.277, and 21.583, preferably having characteristic peaks at 9.385, 14.145, 15.451, 19.709, 20.277, 21.583, and 23.684, more preferably having characteristic peaks at 9.385, 14.145, 15.451, 19.709, 20.277, 21.583, 23.187, 23.684, and 24.366, and most preferably an X-ray powder diffraction pattern represented by a diffraction angle 2*θ* as shown in Fig. 17.

23. The crystal form according to any one of claims 10 to 22, wherein the 2*θ* has an error range of ±0.2.

24. The crystal form according to claims 18 to 22, wherein the chemical ratio of compound (2S)-3-(3-{[(2-{3-[(2S)-2-carboxyl-2-[(3R)-pyrrolidin-3-yl]ethyl]phenoxy}ethyl)({3-[(2S)-2-carboxyl-2-[(3R)-pyrrolidin-3-yl]ethyl]phenyl}methyl)amino]methyl}phenyl)-2-[(3R)-pyrrolidin-3-yl]propionic acid to HCl is 1:1.

25. A pharmaceutical composition, comprising the crystal form according to any one of claims 1 to 5 or 10 to 23, and a pharmaceutically acceptable excipient.

26. A pharmaceutical composition, prepared from the crystal form according to any one of claims 1 to 5 or 10 to 24, and a pharmaceutically acceptable excipient.

27. Use of the pharmaceutically acceptable salt according to claim 6 or 7, or the crystal form according to any one of claims 1 to 5 or 10 to 24, or the pharmaceutical composition according to claim 25 or 26 in the preparation of a medicament for preventing and/or treating a disease or condition associated with elevated LP(a) levels.

28. Use of the pharmaceutically acceptable salt according to claim 6 or 7, or the crystal form according to any one of claims 1 to 5 or 10 to 24, or the pharmaceutical composition according to claim 25 or 26 in the preparation of a medicament for preventing and/or treating a cardiovascular disease.
